# EUROPÄISCHE PATENTSCHRIFT

(11) **EP 1 805 236 B1**
(45) Veröffentlichungstag und Bekanntmachung des Hinweises auf die Patenterteilung: **19.11.2008**
(21) Anmeldenummer: 05798062.5
(22) Anmeldetag: 19.10.2005
(51) Int. Cl.: C08F 220/18, C08F 220/34, C08F 226/06, C08F 220/04

(54) **AMPHOTERE ETHYLMETHACRYLAT-COPOLYMERE UND DEREN VERWENDUNG**
AMPHOTERIC ETHYL METHACRYLATE COPOLYMERS AND USE THEREOF
COPOLYMERES AMPHOTERES DE METHACRYLATE D'ETHYLE ET LEUR UTILISATION

(30) Priorität: 22.10.2004 DE 102004051541
(43) Veröffentlichungstag der Anmeldung: 11.07.2007
(73) Patentinhaber: BASF SE, 67056 Ludwigshafen (DE)
(72) Erfinder: NGUYEN KIM, Son, 69502 Hemsbach (DE); WINTER, Gabi, 67061 Ludwigshafen (DE); LAUBENDER, Matthias, 67105 Schifferstadt (DE)
(74) Vertreter: Fitzner, Uwe
(86) Internationale Anmeldenummer: PCT/EP2005/011241
(87) Internationale Veröffentlichungsnummer: WO 2006/045510

(56) Entgegenhaltungen:
- EP-A- 0 100 890
- EP-A- 0 491 629
- US-A- 3 405 084

## Beschreibung

Die vorliegende Erfindung betrifft amphotere Copolymere, die Ethylmethacrylat, gegebenenfalls eine N-Vinyllactam-Verbindung, wenigstens eine monoethylenisch ungesättigte Carbonsäure und wenigstens eine Verbindung mit α,β-ethylenisch ungesättigter Doppelbindung und wenigstens einer kationogenen und/oder kationischen Gruppe einpolymerisiert enthalten, kosmetische und pharmazeutische Mittel, die solche Copolymere enthalten, Verfahren zur Herstellung sowie die Verwendung dieser Copolymere.

### Stand der Technik

Polymere mit filmbildenden Eigenschaften haben vielfältige Anwendungen im Bereich der Pharmazie und Kosmetik gefunden.

In der Pharmazie dienen sie beispielsweise als Beschichtungsmittel oder Bindemittel für feste Arzneiformen.

In der Kosmetik werden Polymere mit filmbildenden Eigenschaften u. a. zur Festigung, Strukturverbesserung und Formgebung der Haare verwendet. Sie dienen beispielsweise als Conditioner dazu, die Trocken- und Nasskämmbarkeit, das Anfassgefühl, den Glanz und die Erscheinungsform zu verbessern sowie dem Haar antistatische Eigenschaften zu verleihen. Anforderungen, die an filmbildende Polymere für einen Einsatz als Festigerharze gestellt werden, sind z. B. eine starke Festigung (auch bei hoher Luftfeuchtigkeit), hohe Biegesteifigkeit und Elastizität, Auswaschbarkeit vom Haar, Verträglichkeit in der Formulierung und ein angenehmer Griff des damit behandelten Haares. Schwierigkeiten bereitet oft die Bereitstellung von Produkten mit einem komplexen Eigenschaftsprofil. So besteht ein Bedarf an filmbildenden Polymeren für haarkosmetische Mittel, die zur Bildung im Wesentlichen glatter, klebfreier Filme befähigt sind, insbesondere eine gute Festigungswirkung aufweisen und dem Haar gleichzeitig gute sensorisch erfassbare Eigenschaften, wie Elastizität, einen angenehmen Griff und Volumen verleihen.

In Haarsprayformulierungen ist weiterhin eine gute Treibgasverträglichkeit, die Eignung für einen Einsatz in Low-VOC-Formulierungen (VOC = Volatile Organic Compounds), eine gute Versprühbarkeit, eine gute Löslichkeit in Wasser oder wässrig/alkoholischen Lösungsmittelgemischen und eine gute Auswaschbarkeit erwünscht.

Copolymerisate auf (Meth)acrylatbasis, die im alkalischen wasserlöslich sind, werden häufig auf dem Gebiet der Kosmetik als Haarfestiger eingesetzt.

EP-A 491 629 beschreibt eine Aerosol-Zubereitung enthaltend ein neutralisiertes Tetrapolymer bestehend aus a) 4 bis 6 Gew.-% Acrylsäure, b) 42 bis 52 Gew.-% N-Vinylpyrrolidon, c) 15 bis 25 Gew.-% N-Tert.-Butylacrylamid und d) 20 bis 26 Gew.-% Ethylmethacrylat.

DE 2 817 369 beschreibt Copolymerisate, bei denen wenigstens drei der Monomereinheiten Methacrylsäurestruktur haben, wobei die Copolymerisate aus 22 bis 64 Mol-% N,N-Dimethylaminoethylmethacrylat, 13 bis 72 Mol-% Methylmethacrylat, 6 bis 23 Mol-% Methacrylsäure und 0 bis 22 Mol-% mindestens eines N-substituierten Alkyl-(Meth)Acrylamids bestehen.

EP-A 62 002 beschreibt Terpolymere, die durch Copolymerisation von a) 40 bis 60 Gew.-% eines im Alkylteil 1 bis 4 Kohlenstoffatome aufweisenden N-Alkylacrylamides oder N-Alkylmethacrylamides mit b) 35 bis 50 Gew.-% eines C₁-C₄-Hydroxyalkylester oder vorzugsweise C₁-C₄-Alkyl-ester der Acrylsäure oder Methacrylsäure und c) 3 bis 11 Gew.-% einer α,β-ungesättigten Monocarbonsäure oder Dicarbonsäure hergestellt werden.

EP-A 100 890 beschreibt Copolymerisate, erhalten durch radikalische Copolymerisation von 20 bis 75 Gew.teilen mindestens eines C₂-C₂₀-Alkylesters der (Meth)acrylsäure, 5 bis 50 Gewichtsteilen mindestens eines stickstoffhaltigen, neutral reagierenden wasserlöslichen Monomeren, 1 bis 25 Gewichtsteilen mindestens eines kationische Gruppen enthaltenden Monomeren und 1 bis 25 Gewichtsteilen mindestens einer mit a), b) und c) copolymerisierbaren olefinisch ungesättigten C₃-C₅-Carbonsäure, die gemessen in Ethanol bei 25°C einen K-Wert nach Fikentscher von 15 bis 75 aufweisen.

DE 42 23 006 beschreibt Haarbehandlungsmittel, die als Filmbildner Copolymerisate enthalten, die durch Copolymerisieren von (a) 30 bis 80 Gew.-% eines Acryl- oder Methacrylsäureesters, der jeweils als Homopolymerisat eine Glasübergangstemperatur von mehr als 20° C aufweist oder von Gemischen aus Acryl- und Methacrylestern, die bei der Copolymerisation Copolymerisate mit einer Glasübergangstemperatur von mehr als 20°C ergeben, (b) 5 bis 25 Gew.-% Acrylsäure, Methacrylsäure oder deren Mischungen und (c) 10 bis 45 Gew.-% N-Vinylpyrrolidon, N-Vinylcaprolactam oder deren Mischungen in Gegenwart von Radikale bildenden Polymerisationsinitiatoren erhältlich sind, und die in Form der freien Carbonsäuregruppen K-Werte (bestimmt nach H. Fikentscher in 1 gew.-%iger Lösung in Ethanol bei 25°C) von 10 bis 80 haben, dadurch gekennzeichnet, dass man die Copolymerisate nach dem Verfahren der Fällungspolymerisation herstellt.

EP-A 805 169 beschreibt Copolymerisate, die erhältlich sind durch radikalische Polymerisation eines Gemisches aus
a) 30 bis 72 Gew.-% t-Butylacrylat oder t-Butylmethacrylat oder einem Gemisch davon,
b) 10 bis 28 Gew.-% Acrylsäure oder Methacrylsäure oder einem Gemisch davon und
c) 0 bis 60 Gew.-% mindestens eines weiteren radikalisch copolymerisierbaren Monomeren.

EP-A 256 458 beschreibt Copolymerisate zur Verwendung als Haarfixiermittel, erhalten durch radikalische Polymerisation von
a) 20 bis 60 Gew.% Vinylpyrrolidon,
b) 20 bis 60 Gew.% eines am N-Atom mono-oder dialkylierten Acrylamids mit 1 bis 8 C-Atomen im Alkylrest oder ihren Mischungen,
c) 5 bis 60 Gew.% eines Alkyl-oder Hydroxyalkylesters der Acryl-oder Methacrylsäure mit 1 bis 4 C-Atomen im Alkyl- oder 2 bis 4 C-Atomen im Hydroxyalkylrest oder Mischungen dieser Ester oder 3 bis 12 Gew.% Acrylsäure oder Methacrylsaure oder 2 bis 48 Gew.% eines Alkyl-oder Hydroxyalkylesters der Acrylsäure oder Methacrylsäure mit 1 bis 4 C-Atomen im Alkyl oder 2 bis 4 C-Atomen im Hydroxyalkylrest oder Mischungen dieser Ester und 3 bis 12 Gew.% Acrylsäure oder Methacrylsäure, wobei die Gew.% bezogen sind auf das Gesamtgewicht der Monomeren, das in niederen Alkoholen mit 1 bis 4 C-Atomen löslich ist und einen K-Wert von 15 bis 75 aufweist.

US 3,405,084 beschreibt Terpolymere umfassend 20 bis 70 Gew.-% Acrylat, 25 bis 75 Gew.-% N-Vinylpyrrolidon und 3 bis 25 Gew.-% einer Acrylsäure.

EP-A 491 629 beschreibt haarkosmetische Zubereitungen, die als Filmbildner ein Tetrapolymeres aus 4 bis 6 Gew.-% Acrylsäure, 20 bis 26 Gew.-% Ethylmethacrylat, 42 bis 52 Gew.-% N-Vinylpyrrolidon und 15 bis 25 Gew.-% N-tert.-Butylacrylamid enthalten.

US 3,577,517 beschreibt Haarlacke und Aerosol-Zubereitungen, die neutralisierte Lösungspolymerisate aus 5 bis 40 Gew.-% eines (Meth)Acrylsäureesters mit aliphatischen C₈-C₁₈-Alkoholen, 6 bis 35 Gew.-% (Meth)Acrylsäure und 25 bis 89 Gew.-% eines weiteren Vinylmonomeren, ein wasserlösliches organisches Lösungsmittel sowie Treibmittel enthalten.

US 3,927,199 beschreibt Interpolymere aus 30 bis 60 Gew.-% N-Alkyl(Meth)Acrylamid, 12 bis 18 Gew.-% einer ethylenisch ungesättigten Säure, 20 bis 55 Gew.% weiterer Monomere.

EP-A 257 444 beschreibt Terpolymere, erhalten durch radikalische Polymerisation von 20 bis 50 Gew.-% Vinylpyrrolidon, 40 bis 70 Gew.-% tert.-Butylacrylat oder tert.-Butylmethacrylat und 2 bis 15 Gew.-% Acrylsäure oder Methacrylsäure, deren Carboxylgruppen gegebenenfalls zu 5 bis 100 %, bevorzugt 50 bis 90 %, durch ein organisches Amin neutralisiert sind und die einen K-Wert von 10 bis 60 aufweisen.

US 5,045,617 beschreibt zwitterionische Terpolymere bestehend aus einem Vinyllactam, einem Aminoalkylacrylamid oder-acrylat und einer polymerisierbaren Carbonsäure.

### Aufgabe der Erfindung

Strengere Umweltauflagen und wachsendes ökologisches Bewusstsein fordern zunehmend immer geringere Anteile an flüchtigen organischen Komponenten (englisch: volatile organic compounds, VOC) in beispielsweise Haarsprays.

Nach der VOC-Richtlinie (Lösemittel-Richtlinie) sind VOC's flüchtige organische Verbindungen, die bei 293,15 K einen Dampfdruck von 0,01 kPa oder mehr haben und unter den jeweiligen Verwendungsbedingungen eine entsprechende Flüchtigkeit aufweisen.

Der VOC-Gehalt in Haarsprays ist im wesentlichen durch die nicht-wässrigen Lösungsmittel und die Treibmittel gegeben. Daher wurde anstelle von nicht-wässrigen Lösungsmittel verstärkt auf Wasser als Lösungsmittel zurückgegriffen. Dieser Ersatz der organischen Lösungsmitteln bringt aber insbesondere auf dem Gebiet der Haarspray-Formulierungen Probleme mit sich.

So sind Formulierungen der vorgenannten filmbildenden Polymerisate aus dem Stand der Technik, die die entsprechenden VOC-Auflagen erfüllen, beispielsweise nicht oder erst nach weiterer Verdünnung sprühbar und somit nur bedingt für die Verwendung in Haarsprays geeignet. Dies wiederum führt zu Filmen, die mitunter nicht die notwendige mechanische Qualität und somit ungenügende Festigungswirkung und schlechten Halt für das Haar mit sich bringen.

Anforderungen an Haarfestigerharze sind zum Beispiel eine starke Festigung bei hoher Luftfeuchtigkeit, Elastizität, gute Auswaschbarkeit aus dem Haar, Verträglichkeit in der Formulierung, möglichst große Glätte und geringe Klebrigkeit des gebildeten Films und ein angenehmer Griff des damit behandelten Haares. Bei Spray-Formulierungen ist insbesondere auch eine homogene Verteilung kleiner Tröpfchen zur Bildung eines feinen Sprühbildes erwünscht. Schwierigkeiten bereitet oft die Bereitstellung von Produkten mit einem komplexen Eigenschaftsprofil.

Eine Aufgabe der Erfindung bestand darin, für kosmetische Low-VOC-Zubereitungen geeignete Polymere bereitzustellen, die bei Anwendung zu einer starken Festigung bei hoher Luftfeuchtigkeit, guter Auswaschbarkeit aus dem Haar, Verträglichkeit in der Low-VOC-Formulierung, guten rheologischen Eigenschaften wie hoher Biegesteifigkeit und Elastizität sowie möglichst großer Glätte und geringer Klebrigkeit des gebildeten Films und einem angenehmen Griff des damit behandelten Haares führen und bei der Verwendung in einer Low-VOC-Spray-Zubereitung ein gutes Sprühbild ergeben.

Diese Aufgabe wurde gelöst durch die Bereitstellung von Copolymer, erhältlich durch radikalische Polymerisation eines Monomergemisches M enthaltend
a) 30 bis 80 Gew.-% Ethylmethacrylat oder
   30 bis 80 Gew.-% eines Gemisches aus Ethylmethacrylat und wenigstens einer von Ethylmethacrylat unterschiedlichen Verbindung der allgemeinen Formel I 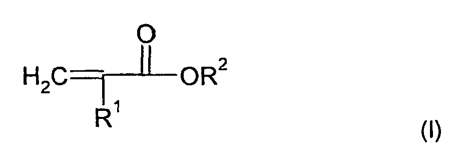 worin
   - R¹: für H oder CH₃ steht,
   - R²: für C₁-C₄-Alkyl steht,
b) 0 bis 40 Gew.-% mindestens einer N-Vinyllactam-Verbindung,
c) 5 bis 35 Gew.-% mindestens einer monoethylenisch ungesättigten Carbonsäure,
d) 0.1 bis 30 Gew.-% mindestens einer Verbindung mit einer radikalisch polymerisierbaren, α,β-ethylenisch ungesättigten Doppelbindung und wenigstens einer kationogenen und/oder kationischen Gruppe pro Molekül,
e) 0 bis 20 Gew.-% gegebenfalls weiterer, von a), b), c) und d) verschiedener, radikalisch polymerisierbarer Monomere,
   wobei sich die Anteile der Komponenten a) bis e) zu 100 Gew.-% addieren und
   wobei das Monomergemisch M wenigstens 20 Gew.-% Ethylmethacrylat enthält.

Das Verhältnis der molaren Gesamtmenge der Carboxyl- und Carboxylat-Gruppen zur molaren Gesamtmenge der kationogenen und kationischen Gruppen befrägt wenigstens 2, besonders bevorzugt mindestens 3, insbesondere mindestens 4 und ganz besonders bevorzugt mindestens 5.

Unter molarer Gesamtmenge der Carboxyl- und Carboxylat-Gruppen wird die Summe aller protonierten und deprotonierten Carboxylgruppen, also aller COOH und COO-Gruppen verstanden.

Unter der molaren Gesamtmenge der kationogenen und kationischen Gruppen wird die Summe aller kationischen und kationogenen, also der in kationische Gruppen überführbaren Gruppen, verstanden.

Im Folgenden werden Verbindungen, die sich von Acrylsäure und Methacrylsäure ableiten können, teilweise verkürzt durch Einfügen der Silbe "(meth)" in die von der Acrylsäure abgeleitete Verbindung bezeichnet.

Unter wasserlöslichen Monomeren und Polymeren werden im Rahmen der vorliegenden Erfindung Monomere und Polymere verstanden, die sich zu mindestens 1 g/l bei 20°C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäßen Copolymere sind im Allgemeinen wasserlöslich.

Die erfindungsgemäßen Copolymere eignen sich in besonders vorteilhafter Weise für einen Einsatz in kosmetischen Mitteln, insbesondere in Haarbehandlungsmitteln. Sie dienen bevorzugt zur Erzeugung elastischer Frisuren bei gleichzeitig starker Festigung. Vorteilhafterweise zeichnen sie sich zudem sowohl durch eine gute Treibgasverträglichkeit, als auch eine gute Löslichkeit in Wasser oder wässrig/alkoholischen Lösungsmittelgemischen aus. Sie lassen sich somit sowohl zu hoch treibgashaltigen Haarsprays (VOC mindestens 85 Gew.-%) als auch zu Formulierungen mit geringen VOC-Werten (im Allgemeinen nicht mehr als 55 Gew.-%, bezogen auf das Gesamtgewicht des Mittels) formulieren. Dabei zeichnen sich die Haarsprayformulierungen in jedem Fall durch eine sehr gute Sprühbarkeit und Auswaschbarkeit aus dem Haar aus.

### Komponente a)

Komponente a) ist Ethylmethacrylat oder eine Mischung von Ethylmethacrylat und mindestens einem weiteren von Ethylmethacrylat unterschiedlichen Verbindung ausgewählt aus der Gruppe bestehend aus Methyl(meth)acrylat, Ethylacrylat, Methylethacrylat, n-Propyl(meth)acrylat, i-Propyl(meth)acrylat, n-Propylethacrylat, Ethylethacrylat, i-Propylethacrylat, n-Butyl(meth)acrylat, n-Butylethacrylat, tert.-Butyl-(meth)acrylat, tert.-Butylethacrylat, i-Butyl(meth)acrylat, i-Butylethacrylat, sec- Butyl(meth)acrylat, Butylethacrylat.

Die erfindungsgemäßen Copolymeren enthalten, bezogen auf das Gesamtgewicht der zur Polymerisation eingesetzten Monomere, mindestens 30 Gew.-%, bevorzugt mindestens 40 Gew.-%, besonders bevorzugt mindestens 50 Gew.-% und höchstens 80 Gew.-%, bevorzugt höchstens 75 Gew.-% und insbesondere höchstens 70 Gew.-% der Komponente a) einpolymerisiert.

Bevorzugt als Komponente a) ist Ethylmethacrylat oder ein Gemisch umfassend oder bestehend aus Ethylmethacrylat und tert-Butylacrylat.

Liegt Ethylmethacrylat in Mischung mit weiteren (Meth)acrylaten als Komponente a) vor, so beträgt der Anteil von Ethylmethacrylat an der zu polymerisierenden Monomerenmischung M aller Monomeren mindestens 20 Gew.-% .

### Komponente b)

Als Monomere b) geeignete N-Vinyllactame sind unsubstituierte N-Vinyllactame und N-Vinyllactamderivate, die z. B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z. B. N-Vinylpyrrolidon, N-Vinylpiperidon, N-Vinyl caprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam und deren Mischungen.

Die zu polymerisierende Monomerenmischung M enthält 0 bis 40 Gew.-% Komponente b).

In einer bevorzugten Ausführungsform der Erfindung enthält die zu polymerisierende Monomerenmischung M keine Komponente b).

In einer weiteren bevorzugten Ausführungsform enthält die zu polymerisierende Monomerenmischung M wenigstens 3, bevorzugt wenigstens 5, besonders bevorzugt wenigstens 10 und höchstens 35, bevorzugt höchstens 30 und besonders bevorzugt höchstens 25 Gew.-% Komponente b).

### Komponente c)

Die erfindungsgemäßen Copolymere enthalten, bezogen auf das Gesamtgewicht der zur Polymerisation eingesetzten Monomere, mindestens 5 Gew.-%, bevorzugt mindestens 10 Gew.-%, besonders bevorzugt mindestens 20 Gew.-% und höchstens 35 Gew.-%, bevorzugt höchstens 30 Gew.-%, besonders bevorzugt höchstens 27 Gew.-% und insbesondere höchstens 25 Gew.-% von wen igstens einer monoethylenisch ungesättigten Carbonsäure als Komponente c) einpolymerisiert.

Zur Herstellung der Copolymere kann die monoethylenisch ungesättigte Carbonsäure c) in teilweise oder vollständig deprotonierter Form eingesetzt werden. Dann leiten sich deren Gegenionen vorzugsweise von Basen ab, wie sie im Folgenden zur Einstellung des pH-Werts bei der Polymerisation oder der erhaltenen Polymerisate beschrieben werden.

Die monoethylenisch ungesättigte Carbonsäure c) ist ausgewählt aus der Gruppe bestehend aus Acrylsäure, Methacrylsäure, Ethacrylsäure, alpha-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Komponenten c) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu c) zählen auch die Salze der zuvor genannten Säuren, insbesondere die Natrium-, Kalium- und Ammoniumsalze. Die Komponenten c) können als solche oder als Mischungen untereinander eingesetzt werden. Die angegebenen Gewichtsanteile beziehen sich sämtlich auf die Säureform.

Vorzugsweise wird Komponente c) ausgewählt aus der Gruppe bestehend aus Acrylsäure, Methacrylsäure, Ethacrylsäure, alpha-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und deren Mischungen, besonders bevorzugt Acrylsäure, Methacrylsäure und deren binäre Mischungen und insbesondere Methacrylsäure.

Ein bevorzugter Gegenstand der Erfindung ist ein erfindungsgemäßes Copolymer wobei Komponente c) der Monomerenmischung M aus Methacrylsäure oder einer Mischung von Methacrylsäure und wenigstens einer weiteren monoethylenisch ungesättigten Carbonsäure besteht.

Insbesondere ist c) Methacrylsäure oder eine Mischung von Methacrylsäure und Acrylsäure.

In einer bevorzugten Ausführrungsform der Erfindung ist das Gewichtsverhältnis von Methacrylsäure zu der wenigstens einen weiteren monoethylenisch ungesättigten Carbonsäure wenigstens größer als 1, bevorzugt größer als 2, besonders bevorzugt größer als 3.

### Komponente d)

Die erfindungsgemäßen Copolymere sind erhältlich durch radikalische Polymerisation einer Monomerenmischung M, die als Komponente d) 0,1 bis 30 Gew.-% mindestens einer Verbindung mit einer radikalisch polymerisierbaren, α,β-ethylenisch ungesättigten Doppelbindung und wenigstens einer kationogenen und/oder kationischen Gruppe pro Molekül enthält.

Bevorzugt handelt es sich bei den kationogenen bzw. kationischen Gruppen der Komponente d) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen. Bevorzugt werden die Verbindungen d) in nicht geladener Form zur Polymerisation eingesetzt. Geeignet ist jedoch auch ein Einsatz in geladener Form. Geladene kationische Gruppen lassen sich z. B. aus den Aminstickstoffen durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure erzeugen.

Vorzugsweise ist die Komponente d) ausgewählt ist unter
d1) Estern α,β-ethylenisch ungesättigter Mono- und Dicarbonsäuren mit Aminoalkoholen, welche am Aminstickstoff mono- oder dialkyliert sein können,
d2) Amiden α,β-ethylenisch ungesättigter Mono- und Dicarbonsäuren mit Diaminen, welche mindestens eine primäre oder sekundäre Aminogruppe aufweisen,
d3) N,N-Diallylamin,
d4) N,N-Diallyl-N-alkylaminen und deren Derivaten,
d5) vinyl- und allylsubstituierten Stickstoffheterocyclen,
d6) vinyl- und allylsubstituierten heteroaromatischen Verbindungen und Mischungen davon.

Geeignet als Verbindungen d) sind d1) die Ester von α,β-ethylenisch ungesättigten Mono- und Dicarbonsäuren mit Am inoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Besonders bevorzugt als Verbindungen d) sind N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Insbesondere werden als Verbindung d) N-(tert.-Butyl)aminoethylacrylat und N-(tert.-Butyl)aminoethylmethacrylat eingesetzt.

Geeignete Monomere d) sind weiterhin d2) die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen. Bevorzugt werden als Monomere e) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid und N-[4-(dimethylamino)cyclohexyl]-methacrylamid eingesetzt.

Besonders bevorzugt werden N-[3-(dimethyl-amino)propyl]acrylamid und/oder N-[3-(dimethylamino)propyl]methacrylamid eingesetzt.

Geeignete Monomere d) sind weiterhin d3) N,N-Diallylamine und d4) N,N-Diallyl-N-alkylamine und deren Säureadditionssalze. Alkyl steht dabei vorzugsweise für C₁-C₂₄-Alkyl. Bevorzugt ist z. B. N,N-Diallyl-N-methylamin.

Geeignete Monomere d) sind weiterhin d5) vinyl- und allylsubstituierte Stickstoffheterocyclen, wie N-Vinylimidazol, N-Vinylimidazol-Derivate, z. B. N-Vinyl-2-methylimidazol, vinyl- und allylsubstituierte heteroaromatische Verbindungen, wie 2- und 4-Vinylpyridin, 2- und 4-Allylpyridin, und die Salze davon.

Geeignete Monomere d) sind auch N-Vinylimidazole der allgemeinen Formel (II), worin R¹ bis R³ für Wasserstoff, C₁-C₄-Alkyl oder Phenyl steht

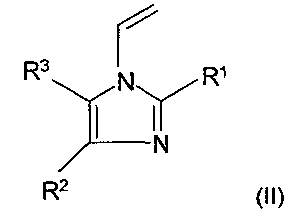

Beispiele für Verbindungen der allgemeinen Formel (II) sind folgender Tabelle 1 zu entnehmen:

**Tabelle 1**

| R¹ | R² | R³ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Besonders bevorzugt sind die Verbindungen der Komponente d) ausgewählt unter N-(tert.-Butylamino)ethyl(meth)acrylat, N,N-Dimethylaminoethyl(meth)acrylat, N-[3-(dimethylamino)propyl](meth)acrylamid, Vinylimidazol und deren Mischungen.

Die erfindungsgemäßen Copolymere enthalten wenigstens 0.1 Gew.-%, bevorzugt wenigstens 1 Gew.-%, besonders bevorzugt wenigstens 2 Gew.-% und insbesondere wenigstens 3 Gew.-% und höchstens 30 Gew.-%, bevorzugt höchstens 20 Gew.-%, besonders bevorzugt höchstens 15 Gew.-% und insbesondere höchstens 10 Gew.-%, Monomer d) bezogen auf das Gesamtgewicht der zur Polymerisation eingesetzten Monomere, einpolymerisiert.

Eine bevorzugte Ausführungsform der Erfindung sind Copolymere erhältlich durch radikalische Polymerisation eines Monomergemisches M enthaltend
30 bis 70 Gew.-% Komponente a),
10 bis 40 Gew.-% Komponente b),
10 bis 25 Gew.-% Komponente c),
0,5 bis 6 Gew.-% Komponente d).

Eine weitere bevorzugte Ausführungsform der Erfindung sind Copolymere erh ältlich durch radikalische Polymerisation eines Monomergemisches M enthaltend
50 bis 80 Gew.-% Komponente a),
0 Gew.-% Komponente b),
15 bis 35 Gew.-% Komponente c),
0,5 bis 12 Gew.-% Komponente d).

Die erfindungsgemäßen Copolymere können zusätzlich wenigstens ein von den Komponenten a) bis d) verschiedenes, damit copolymerisierbares Monomer e) einpolymerisiert enthalten.

Vorzugsweise ist die Komponente e) ausgewählt unter Estern α,β-ethylenisch ungesättigter Mono- und Dicarbonsäuren mit C₁-C₃₀-Alkanolen und C₁-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen, N-Vinylamiden gesättigter Monocarbonsäuren, primären Amiden α,β-ethylenisch ungesättigter Monocarbonsäuren und deren N-Alkyl- und N,N-Dialkylderivaten, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

Als Monomere e) geeignete N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid. In einer bevorzugten Ausführung enthalten die erfindungsgemäßen Copolymere keine N-Vinylamidverbindungen einpolymerisiert.

Geeignete zusätzliche Monomere e) sind weiterhin 2-Hydroxyethylacrylat, 2-Hydroxyethylmethacrylat, 2-Hydroxyethylethacrylat, 2-Hydroxypropylacryl at, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete zusätzliche Monomere e) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Geeignete Monomere e) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.

Bevorzugt als Komponente e) sind Polyetheracrylate der allgemeinen Formel III 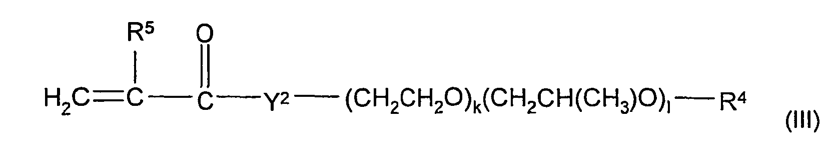 worin
die Reihenfolge der Alkylenoxideinheiten beliebig ist,
- k und l: unabhängig voneinander für eine ganze Zahl von 0 bis 1000 stehen, wobei die Summe aus k und lmindestens 5 beträgt,
- R⁴: für Wasserstoff, C₁-C₃₀-Alkyl oder C₅-C₈-Cycloalkyl steht,
- R⁵: für Wasserstoff oder C₁-C₈-Alkyl steht,
- Y²: für O oder NR⁶ steht, wobei R⁶ für Wasserstoff, C₁-C₃₀-Alkyl oder C₅-C₈-Cycloalkyl steht,

Bevorzugt steht k für eine ganze Zahl von 1 bis 500, insbesondere 3 bis 250. Bevorzugt steht I für eine ganze Zahl von 0 bis 100.

Bevorzugt steht R⁵ für Wasserstoff, Methyl, Ethyl, n-Propyl, Isopropyl, n-Butyl, sek.-Butyl, tert.-Butyl, n-Pentyl oder n-Hexyl, insbesondere für Wasserstoff, Methyl oder Ethyl.

Vorzugsweise steht R⁴ in der Formel II für Wasserstoff, Methyl, Ethyl, n-Propyl, Isopropyl, n-Butyl, sek.-Butyl, n-Pentyl, n-Hexyl, Octyl, 2-Ethylhexyl, Decyl, Lauryl, Pal mityl oder Stearyl.

Vorzugsweise steht Y² in der Formel III für O oder NH.

Geeignete Polyetheracrylate e) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁴-OH hergestellt werden. Die Alkylenoxide können einzeln, alternierend nacheinander oder als Mischung eingesetzt werden. Die Polyetheracrylate e) können allein oder in Mischungen zur Herstellung der erfindungsgemäß eingesetzten Polymere verwendet werden.

Geeignete zusätzliche Monomere e) sind die von Komponente a) verschiedenen Ester von α,β-ethylenisch ungesättigten Mono- und Dicarbonsäuren wie beispielsweise Methyl(meth)acrylat, Ethylacrylat, Methylethacrylat, n-Propyl(meth)acrylat, i-Propyl(meth)-acrylat, n-Propylethacrylat, Ethylethacrylat, i-Propylethacrylat, n-Butyl(meth)acrylat, n-Butylethacrylat, tert.-Butyl(meth)acrylat, tert.-Butylethacrylat, i-Butyl(meth)acrylat, i-Butylethacrylat, sec- Butyl(meth)acrylat, Butlyethacrylat, 2-Pentyl(meth)acrylat, 3-Pentyl(meth)acrylat, Isopentylacrylat, Neopentylacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl)(meth)acrylat, Tridecyl(meth)acrylat, Myristyl(meth)-acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl-(meth)acrylat, Cerotinyl(meth)acrylat, Melissinyl(meth)acrylat, Palmitoleinyl(meth)-acrylat, Oleyl(meth)acrylat, Linolyl(meth)acrylat, Linolenyl(meth)acrylat, Stearyl-(meth)acrylat, Lauryl(meth)acrylat, Phenoxyethylacrylat, t-Butylcyclohexylacrylat, Cyclohexyl(meth)acrylat, Ureido(meth)acrylat, Tetrahydrofurfuryl(meth)acrylat und Mischungen davon.

Bevorzugte Monomere e) sind die Ester α,β-ethylenisch ungesättigter Mono- und Dicarbonsäuren mit C₁-C₄-Alkanolen.

Geeignete zusätzliche Monomere e) sind weiterhin N-(n-Butyl)methacrylamid, N-(sek.-Butyl)methacrylamid, N-(tert.-Butyl)methacrylamid, N-(n-Pentyl)(meth)acrylamid, N-(n-Hexyl)(meth)acrylamid, N-(n-Heptyl)(meth)acrylamid, N-(n-Octyl)(meth)acrylamid, N-(tert.-Octyl)(meth)acrylamid N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arrachinyl(meth)acrylamid, N-Behenyl(meth)acrylamid, N-Lignocerenyl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid.

Geeignete zusätzliche Monomere e) sind weiterhin Vinylacetat, Vinylpropionat, Vinylbutyrat und Mischungen davon.

Geeignete zusätzliche Monomere e) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere e) können einzeln oder in Form von beliebigen Mischungen eingesetzt werden.

Die erfindungsgemäßen Copolymere enthalten höchstens 10 Gew.-%, besonders bevorzugt höchstens 7 Gew.-%, insbesondere höchstens 5 Gew.-%, bezogen auf das Gesamtgewicht der zur Polymerisation eingesetzten Monomere, wenigstens ein Monomer e) einpolymerisiert.

Wenn ein Monomer e) eingesetzt wird, dann vorzugsweise in einer Menge von mindestens 0.5 Gew.-%, besonders bevorzugt mindestens 2 Gew.-% und insbesondere mindestens 3 Gew.-%.

### Vernetzer f)

Die erfindungsgemäßen Copolymere können gewünschtenfalls wenigstens einen Vernetzer, d.h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 3 Gew.-% , besonders bevorzugt 0,1 bis 2 Gew.-%, bezogen auf das Gesamtgewicht der zur Polymerisation eingesetzten Monomere, verwendet.

Geeignete Vernetzer f) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylen oxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer f) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer f) sind Ester ungesättigter Carbonsäuren mit den oben beschriebenen mehrwertigen Alkoholen, beispielsweise der Ölsäure, Crotonsäure, Zimtsäure oder 10-Undecensäure.

Weitere geeignete Vernetzer f) sind Urethandiacrylate und Urethanpolyacryl ate, wie sie z. B. unter der Bezeichnung Laromer^{®} kommerziell erhältlich sind.

Geeignet als Vernetzer f) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer f) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer f) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer f) sind Divinyldioxan, Tetraallylsilan oder Tetravinylsilan.

Selbstverständlich können auch Mischungen der vorgenannten Verbindungen f) eingesetzt werden. Vorzugsweise werden wasserlösliche Vernetzer f) eingesetzt.

Besonders bevorzugt eingesetzte Vernetzer f) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer f) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

In einer bevorzugten Ausführungsform sind erfindungsgemäße Copolymere erhältlich durch radikalische Polymerisation von a) Ethylmethacrylat, b) N-Vinylpyrrolidon oder N-Vinylcaprolactam oder deren Mischungen, c) Methacrylsäure oder einer Mischung aus Methacrylsäure und Acrylsäure und Komponente d) ausgewählt aus der Gruppe bestehend aus N-[3-(dimethylamino)propyl](meth)acrylamid, N-(tert.-Butyl)aminoethylmethacrylat, Vinylimidazol und deren Mischungen.

### Herstellung der Copolymere

Die Herstellung der erfindungsgemäßen Copolymere kann z. B. durch Lösungs-, Fällungs-, Suspensions- oder Emulsionspolymerisation erfolgen. Derartige Verfahren sind dem Fachmann prinzipiell bekannt. Bevorzugt ist die Herstellung durch Lösungspolymerisation.

Bevorzugte Lösemittel zur Polymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan.

Besonders bevorzugt ist die Polymerisation in Wasser oder einem Wasser/AlkoholGemisch, beispielsweise in einem Wasser/Ethanol-Gemisch.

Die Polymerisation kann prinzipiell bei dem durch die eingesetzten Monomere resultierenden pH-Wert erfolgen. Wird zur Polymerisation wenigstens ein N-Vinyllactam eingesetzt (= Komponente b)), so wird der pH-Wert des Polymerisationsmediums vorzugsweise auf einen Wert von 5 bis 8, bevorzugt 6.5 bis 7.5, eingestellt. Es ist vorteilhaft, den pH-Wert während der Polymerisation dann in diesem Bereich zu halten. Zur Einstellung des pH-Werts vor, während oder nach der Polymerisation eignen sich prinzipiell alle anorganischen oder organischen Basen (und gegebenenfalls Säuren), insbesondere solche, die außer einer etwaigen Salzbildung keine Reaktion mit den Monomeren eingehen. Geeignete Basen sind z. B. Alkali- und Erdalkalihydroxide, Ammoniak sowie primäre, sekundäre und tertiäre Amine, wie Triethylamin, sowie Aminoalkohole, wie Triethanolamin, Methyldiethanolamin, Dimethylethanolamin oder 2-Amino-2-methylpropanol. Bevorzugt wird zur Einstellung des pH-Werts wenigstens ein tertiären Amin, das insbesondere ausgewählt ist unter N,N-Dimethylethanolamin, N-Methyldiethanolamin, Triethanolamin und Mischungen davon, eingesetzt. Wird zur Polymerisation wenigstens ein N-Vinyllactam eingesetzt (= Kompon ente b)), so wird der pH-Wert des Polymerisationsmediums vorzugsweise mit N,N-Dimethylethanolamin eingestellt.

Die Polymerisationstemperaturen liegen vorzugsweise in einem Bereich von etwa 30 bis 120°C, besonders bevorzugt 40 bis 100°C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Copolymerisation können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azobis(2-amidinopropan)hydrochloride (V50 von Wako Pure Chemicals Industries, Ltd.), oder 2,2'-Azobis(2-methyl-butyronitril). Geeignet sind auch Initiatormischungen oder Redox-Initiator-Systeme, wie z. B. Ascorbinsäure/Eisen(II)-sulfat/Natriumperoxodisulfat, tert.-Butylhydroperoxid/Natriumdisulfit, tert.-Butylhydroperoxid/Natriumhydroxymethansulfinat, H₂O₂/Cu^{l}.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, T-hioglycolsäure oder Dodecylmercaptan sowie Tribromchlormethan oder andere Verbindungen, die regelnd auf das Molekulargewicht der erhaltenen Polymerisate wirken, ei ngesetzt werden. Ein bevorzugter Regler ist Cystein.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Wird bei der Herstellung der Polymere ein organisches Lösungsmittel eingesetzt, so kann dieses durch übliche, dem Fachmann bekannte Verfahren, z. B. durch Destillation bei vermindertem Druck, entfernt werden.

Enthält die Monomerenmischung M N-Vinyllactame (Komponente b)), so ist es vorteilhaft, während der Polymerisation den pH-Wert auf einen Wert im Bereich 5 bis 8, bevorzugt 6.5 bis 7.5 einzustellen und nach abgeschlossener Polymerisation den pH-Wert auf einen Wert im Bereich von 5 bis 7, bevorzugt 5.5 bis 6.5 einzustellen und anschließend eine Wasserdampfdestillation der Polymerisationslösung du rchzuführen.

Ein weiterer Gegenstand der Erfindung ist also ein Verfahren zur Herstellung der erfindungsgemäßen Copolymere umfassend die Schritte
1) radikalische Polymerisation des Monomergemisches M enthaltend die Komponenten a), b), c), d) und gegebenenfalls e) bei einem pH-Wert im Bereich von 5 bis 8, bevorzugt 6,5 bis 7,5,
2) Einstellen des pH-Wertes nach abgeschlossener Polymerisation auf einen Wert im Bereich von 5 bis 7, bevorzugt 5,5 bis 6,5,
3) Wasserdampf-Destillation der Polymerisationslösung,
4) gegebenenfalls Einstellung des pH-Wertes der destillierten Polymerisationslösung auf einen Wert im Bereich von 7,5 bis 9,0, bevorzugt 8,0 bis 8,5 nach der Wasserdampfdestillation.

Die Polymerisation gilt als abgeschlossen, wenn der Anteil an nicht polymerisierten Monomeren (=Restmonomere) in der Polymerisationslösung höchstens 1 Gew.-%, bevorzugt höchstens 0,1 Gew.%, besonders bevorzugt höchstens 0,01 Gew.-% bezogen auf das Gesamtgewicht der zur Polymerisation eingesetzten Monomere beträgt.

### Neutralisation

Darüberhinaus können die vorliegenden Polymerisate teilweise oder vollständig neutralisiert werden. Insbesondere zur Verwendung der Polymerisate in haarkosmetischen Zubereitungen ist eine teilweise oder vollständige Neutralisation vorteilhaft, In bevorzugten Ausführungsformen sind die Polymere beispielsweise zu mindestens 10, bevorzugt zu mindestens 30, weiter bevorzugt zu mindestens 40, besonders bevorzugt zu mindestens 50, ganz besonders bevorzugt zu mindestens 70 und insbesondere zu mindestens 95 % neutralisiert.

In einer ganz besonders bevorzugten Ausführungsform sind die Polymere zu mindestens 99% neutralisiert. Am meisten bevorzugt ist die Neutralisation zu mindestens 100%.

Es ist weiterhin vorteilhaft, wenn das Neutralisationsmittel in mehr als äquivalenter Menge zugesetzt wird, wobei unter äquivalenter Menge die Menge verstanden wird, die benötigt wird, um alle neutralisierbaren Gruppen der Polymere zu neutralisieren.

Die Neutralisation kann auch erfolgen mit
- einem Mono-, Di- oder Trialkanolamin mit 2 bis 5 Kohlenstoffatomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,
- einem Alkandiolamin mit 2 bis 5 Kohlenstoffatomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 Kohlenstoffatomen, beispielsweise N,N-Diethylpropylamin oder 3-Diethylamino-1-propylamin.

Als Alkalimetallhydroxide eignen sich zur Neutralisation vor allem Natrium-, oder Kalium- sowie Ammoniumhydroxid.

Häufig werden gute Neutralisationsergebnisse mit 2-Amino-2-methylpropanol, Triisopropanolamin, 2-Amino-2-ethylpropan-1,3-diol, N,N-Dimethylaminoethanol oder 3-Diethylamino-1-propylamin erhalten.

Zur Neutralisation der Polymere in den erfindungsgemäßen Zubereitungen und Mitteln sind inbesondere Aminogruppen enthaltende Silikonpolymere geeignet. Geeignete Aminogruppen enthaltende Silikonpolymere sind beispielsweise die Silikon-Aminopolyalkylenoxid-Blockcopolymere der WO 97/32917, die Produkte Silsoft^{®}A-843 (Dimethicone Bisamino Hydroxypropyl Copolyol) und Silsoft^{®}A-858 (Trimethylsilyl A-modimethicone Copolymer) (beide Fa. Witco). Weiterhin sind auch die Neutralisations-polymere der EP-A 1035144 und insbesondere die silikonhaltigen Neutralisationspoly-mere gemäß Anspruch 12 der EP-A 1035144 geeignet.

### Trocknung

Die erhaltenen flüssigen Polymerzusammensetzungen können durch verschiedene Trocknungsverfahren, wie z. B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

### Kosmetische und pharmazeutische Mittel

Die zuvor beschriebenen Copolymere eignen sich hervorragend zur Herstellung kosmetischer und pharmazeutischer Mittel. Sie dienen dabei z. B. als polymere Filmbildner in Zubereitungen für die Körperpflege, was die Anwendung in kosmetischen Zubereitungen für keratinöse Oberflächen wie Haut, Haar, Nägel sowie a uch Mundpflegepräparate beinhaltet. Sie sind universell in den verschiedensten kosmetischen Zubereitungen einsetzbar und einformulierbar und mit den üblichen Komponenten verträglich. Insbesondere eignen sich die erfindungsgemäßen Copolymere zur Herstellung haarkosmetischer Mittel. Gegenüber üblichen, aus dem Stand der Technik bekannten Polymeren eignen sie sich vorteilhaft zur Erzeugung elastischer Frisuren bei gleichzeitig starker Festigung (auch bei hoher Luftfeuchtigkeit). Die erfindung sgemäßen Copolymere zeichnen sich zudem durch eine gute Treibgasverträglichkeit, eine gute Löslichkeit in Wasser oder wässrig/alkoholischen Lösungsmittelgemischen, die Eignung für einen Einsatz in Low-VOC-Formulierungen und eine gute Auswaschbarkeit aus. Zudem haben sie in der Regel auch gute konditionierende Eigenschaften, d. h. sie verbessern damit behandeltes Haar in seinen sensorisch erfassbaren Eigenschaften wie Griff, Volumen, Handhabbarkeit usw. Haarsprayformulierungen auf Basis der erfindungsgemäßen Copolymere zeichnen sich durch gute rheologische Eigenschaften und eine gute Sprühbarkeit aus.

### Kosmetisch oder pharmazeutisch akzeptabler Träger B)

Die erfindungsgemäßen Mittel weisen einen kosmetisch oder pharmazeutisch akzeptablen Träger B) auf, der ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolm onolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Methanol, n-Propanol, iso-Propanol, Propylenglykol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Copolymere insbesondere als Zusatzstoffe für Haar- und Hautkosmetika.

Vorzugsweise liegen die erfindungsgemäßen Mittel in Form eines Sprays, Gels, Schaums, einer Salbe, Creme, Emulsion, Suspension, Lotion, Milch oder Paste vor. Gewünschtenfalls können auch Liposomen oder Mikrosphären eingesetzt werden.

In einer bevorzugten Ausführungsform der Erfindung weisen die Mittel einen Anteil an flüchtigen organischen Komponenten von höchstens 80 Gew.-%, bevorzugt höchstens 55 Gew.-% und insbesondere höchstens 30 Gew.-% auf. Ein bevorzugter Gegenstand sind somit Mittel, die dem low-VOC-Standard, also VOC-80- und VOC-55-Standard entsprechen.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer (= Komponente A), wenigstens einen wie vorstehend definierten Träger B) und wenigstens einen davon verschiedenen Bestandteil, der ausgewählt ist unter kosmetisch aktiven Wirkstoffen, Emulgato ren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollentien und Weichmachern.

### Verdickungsmittel

Übliche Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellu lose oder Hydroxycarboxymethylcellulose, Fettalkohole, Monoglyceride und Fettsäuren, Polyvinylalkohol und Polyvinylpyrrolidon. Bevorzugt werden nichtionische Verdicker eingesetzt.

### Kosmetisch und/oder dermatologisch aktive Wirkstoffe

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc.

Geeignete Lichtfilterwirkstoffe sind Stoffe, die UV-Strahlen im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Meth oxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid.

Beispielsweise sind als UV-Lichtschutzfilter zu nennen:

| Nr. | Stoff | CAS-Nr. |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon(Oxybenzon um) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) un d ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | Menthyl-o-aminobenzoate oder:5-Methyl-2-(1-methylethyl)-2-aminobenzoate | 134-09-8 |
| 20 | Glyceryl p-aminobenzoat oder 4-Aminobenzoesäure-1-glyceryl-ester | 136-44-7 |
| 21 | 2,2'-Dihydroxy-4-methoxybenzophenon (Dioxybenzone) | 131-53-3 |
| 22 | 2-Hydroxy-4-methoxy-4-methylbenzophenon (Mexenon) | 1641-17-4 |
| 23 | Triethanolamin Salicylat | 2174-16-5 |
| 24 | Dimethoxyphenylglyoxalsäure oder:3,4-dimethoxy-phenyl-glyoxal-saures Natrium | 4732-70-1 |
| 25 | 3-(4'Sulfo)benzyliden-bornan-2-on und seine Salze | 56039-58-8 |
| 26 | 2,2',4,4'-Tetrahydroxybenzophenon | 131-55-5 |
| 27 | 2,2'-Methylen-bis-[6(2H-benzotriazol-2-yl)-4-(1,1,3,3,-tetramethylbutyl)phenol] | 103597-45-1 |
| 28 | 2,2'-(1,4-Phenylen)-bis-1H-benzimidazol-4,6-disulfonsäure, Na-Salz | 180898-37-7 |
| 29 | 2,4-bis-[4-(2-Ethylhexyloxy)-2-hydroxy]phenyl-6-(4-methoxyphenyl)-(1,3,5) triazin | 187393-00-6 |
| 30 | 3-(4-Methylbenzyliden)-campher | 36861-47-9 |
| 31 | 4-Bis(polyethoxy)paraaminobenzoesäurepolyethoxyethylester | 113010-52-9 |
| 32 | 2,4-Dihydroxybenzophenon | 131-56-6 |
| 33 | 2,2'-Dihydroxy-4,4'-dimethoxybenzophenon-5,5'-dinatriumsulfonat | 3121-60-6 |

Weitere kombinierbare Lichtschutzmittel sind u.a. folgende Verbindungen:

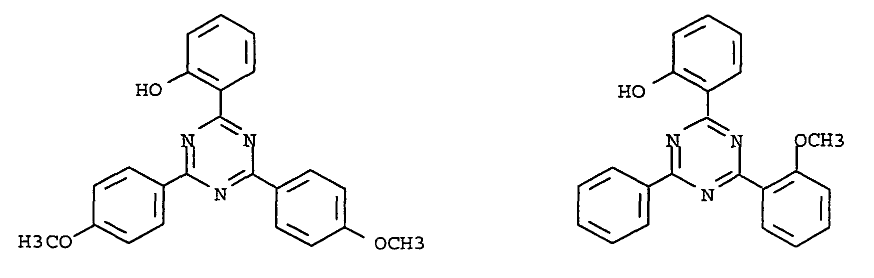

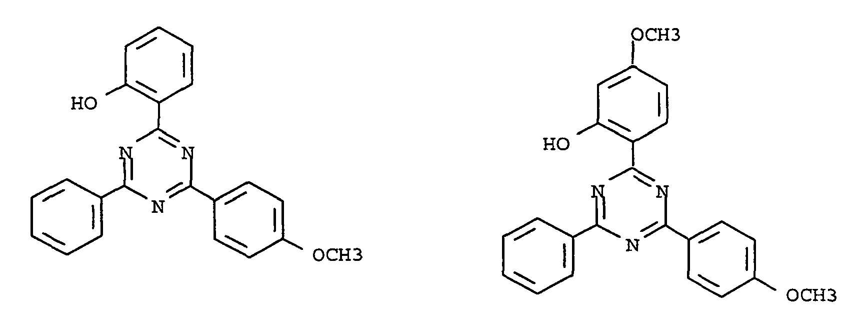

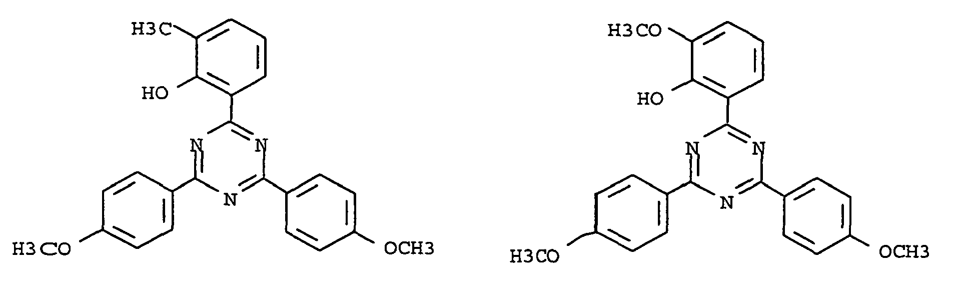

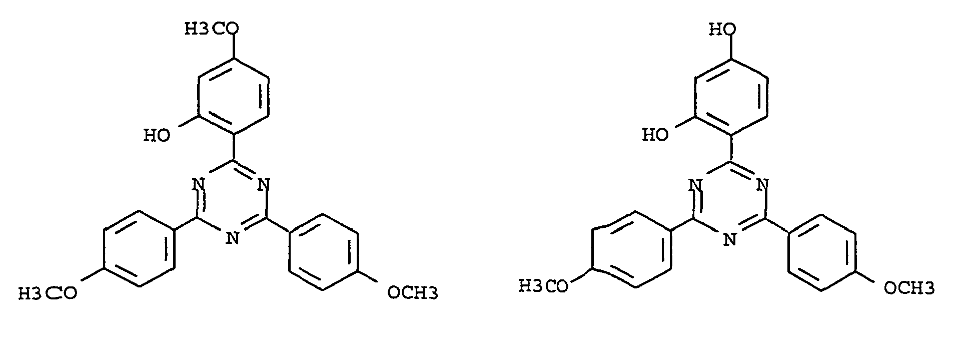

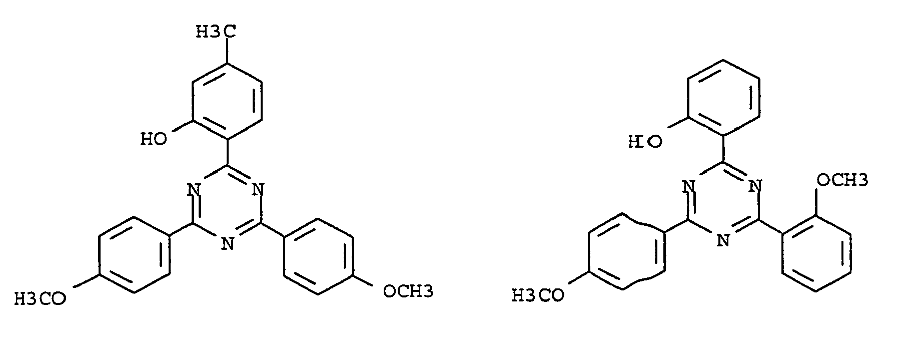

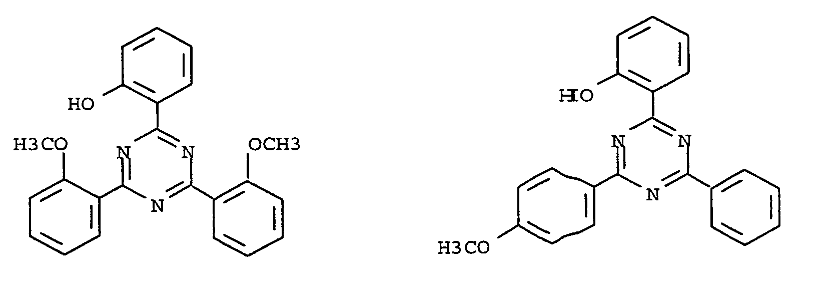

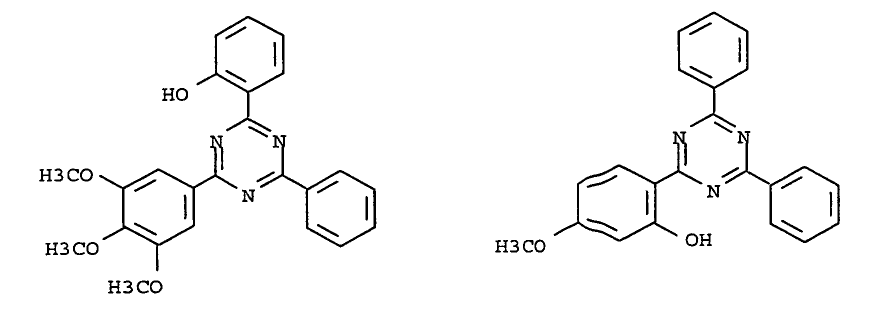

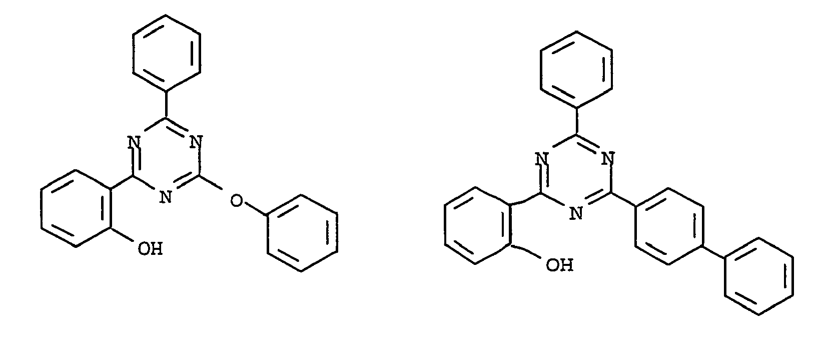

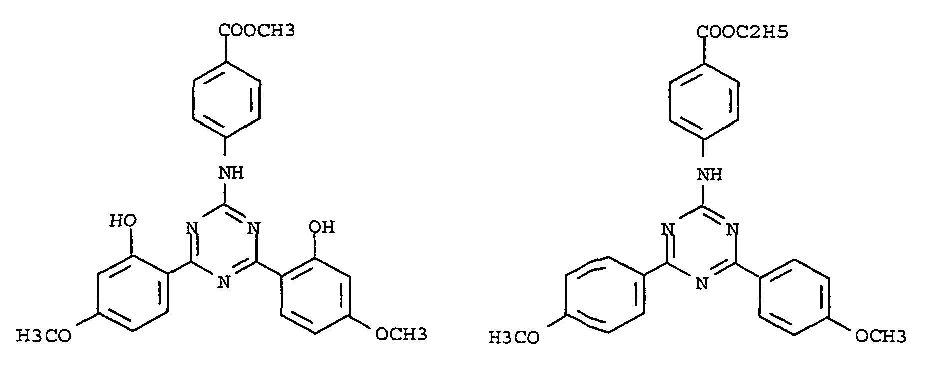

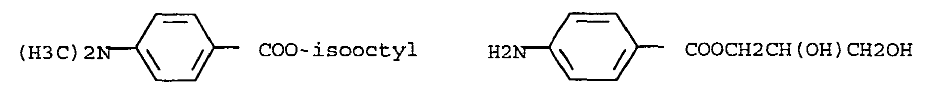

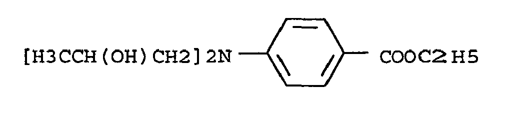

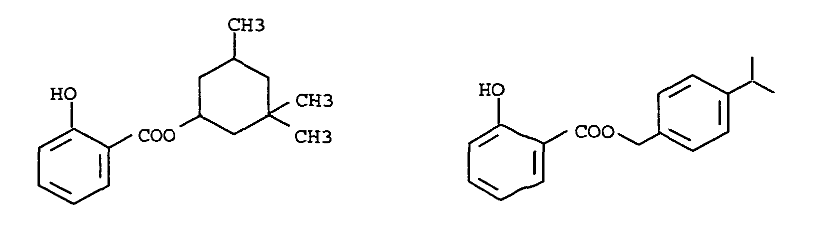

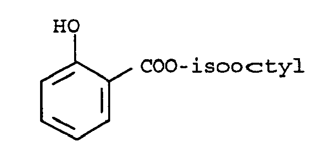

Geeignet sind weiterhin UV-Strahlen abhaltende Pigmente, wie Titandioxid, Talkum und Zinkoxid.

Zur Verwendung in den erfindungsgemäßen Zubereitungen geeignete Lichtschutzmittel sind weiterhin die in der EP-A 1 084 696 in den Absätzen [0036] bis [0053] genannten Verbindungen, auf die hier vollumfänglich Bezug genommen wird.

Die Aufzählung der genannten UV-Lichtschutz-Filter, die in den erfindungsgemäßen Zubereitungen verwendet werden können, soll selbstverständlich nicht limitierend sein.

### Keimhemmende Mittel

Weiterhin können in den erfindungsgemäßen Zubereitungen auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid).

Quartäre Ammonium-Verbindungen sind prinzipiell ebenfalls geeignet, werden jedoch bevorzugt für desinfizierende Seifen und Waschlotionen verwendet.

Auch zahlreiche Riechstoffe haben antimikrobielle Eigenschaften. Spezielle Kombinationen mit besonderer Wirksamkeit gegenüber grampositiven Bakterien werden für die Komposition sog. Deoparfums eingesetzt.

Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.

Die antibakteriell wirksamen Stoffe werden in der Regel in Konzentrationen von ca. 0,1 bis 0,3 Gew.-% eingesetzt.

Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc.

Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc.

Geeignete keratolytisch und keratoplastisch wirkende Stoffe sind z. B. Salicylsäure, Kalziumthioglykolat, Thioglykolsäure und ihre Salze, Schwefel, etc.

Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc..

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten.

Bevorzugt sind Mittel die zusätzlich wenigstens ein nichtionisches, ein anionisches oder ein ampholytisches Polymer enthalten.

Als zusätzliche Polymere bevorzugte anionische Polymere sind beispielsweise Homo- und Copolymerisate von Acrylsäure und Methacrylsäure und deren Salze. Dazu zählen auch vernetzte Polymere der Acrylsäure, wie sie unter dem INCl-Namen Carbomer erhältlich sind. Derartige vernetzte Homopolymere der Acrylsäure sind beispielsweise kommerziell unter dem Namen Carbopol^{®} von der Firma BF GOODRICH erhältlich. Bevorzugt sind auch hydrophob modifizierte vernetzte Polyacrylat Polymere, wie Carbopol^{®} Ultrez 21 von der Firma Noveon.

Weitere Beispiele für geeignete zusätzliche anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe.

Bevorzugt sind weiterhin Mittel, die zusätzlich ein Polyurethan als anionisches Polymer enthalten.

Besonders geeignet als zusätzliche Polymere sind die in der DE 4225045 A1 beschriebenen wasserlöslichen oder wasserdispergierbaren Polyurethane, auf die hier in vollem Umfang Bezug genommen wird. Insbesondere geeignet ist Luviset^{®}P.U.R. (BASF). Weiterhin besonders bevorzugt sind silikonhaltige Polyurethane, wie sie in der DE 19807908 A1 beschrieben sind, auf die hier in vollem Umfang Bezug genommen wird. Insbesondere geeignet ist Luviset^{®}Si-P.U.R. (BASF).

Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn^{®} von der Firma Rohm und Haas erhältlich sind. Besonders geeignete Polymere sind weiterhin Copolymere aus t-Butylacrylat, Ethylacrylat, Methacrylsäure (z. B. Luvimer^{®}100P), Copolymere aus Ethylacrylat und Methacrylsäure (z. B. Luviumer^{®} MAE), Copolymere aus N-tert.-Butylacrylamid, Ethylacrylat, Acrylsäure (Ultrahold^{®}8, strong), Copolymere aus Vinylacetat, Crotonsäure und gegebenenfalls weitere Vinylester (z. B. Luviset^{®} Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol^{®}VBM), Copolymere von Acrylsäure und Methacrylsäure mit hydro phoben Monomeren, wie z. B. C₄-C₃₀-Alkylester der Meth(acrylsäure), C₄-C₃₀-Alkylvinylester, C₄-C₃₀-Alkylvinylether und Hyaluronsäure. Beispiele für an ionische Polymere sind weiterhin Vinylacetat/Crotonsäure-Copolymere, wie sie beispielsweise unter den Bezeichnungen Resyn^{®} (National Starch) und Gafset^{®} (GAF) im Handel sind und Vinylpyrrolidon/Vinylacrylat-Copolymere, erhältlich beispielsweise unter dem Warenzeichen Luviflex^{®} (BASF). Weitere geeignete Polymere sind das unter der Bezeichnung Luviflex^{®}VBM-35 (BASF) erhältliche Vinylpyrrolidon/Acrylat-Terpolymer und Natriumsulfonat-haltige Polyamide oder Natriumsulfonat-haltige Polyester.

Weiterhin umfasst die Gruppe der geeigneten anionischen Polymere beispielhaft Balance^{®} CR (National Starch; Acrylate Copolymer), Balance^{®} 0/55 (National Starch; Acrylate Copolymer), Balance^{®} 47 (National Starch; Octylacrylamid/Acrylate/Butylaminoethylmethacrylate-Copolymer), Aquaflex^{®} FX 64 (ISP; Isobutylen/Ethylmaleimid/Hydroxyethylmaleimid-Copolymer), Aquaflex^{®} SF-40 (ISP / National Starch; VP/Vinyl Caprolactam/DMAPA Acrylate Copolymer), Allianz^{®} LT-120 (ISP; Rohm & Haas; Acrylat/C1-2 Succinat/Hydroxyacrylat Copolymer), Aquarez^{®} H S (Eastman; Polyester-1), Diaformer^{®} Z-400 (Clariant; Methacryloylethylbetain/Methacrylat-Copolymer), Diaformer^{®} Z-711 (Clariant; Methacryloylethyl N-oxid/Methacrylat-Copolymer), Diaformer^{®} Z-712 (Clariant; Methacryloylethyl N-oxide/Methacrylat-Copolymer), Omnirez^{®} 2000 (ISP; Monoethylester von Poly(Methylvinylether/Maleinsäure in Ethanol), Amphomer^{®} HC (National Starch; Acrylat/ Octylacrylamid-Copolymer), Amphomer^{®} 28-4910 (National Starch; Octylacrylamid/Acrylat/Butylaminoethylmethacrylat-Copolymer), Advantage^{®} HC 37 (ISP; Terpolymer aus Vinylcaprolactam/Vinylpyrrolidon/Dimethylaminoethylmethacrylat), Advantage^{®} LC55 und LC80 oder LC A und LC E, Advantage^{®} Plus (ISP; VA/Butyl Maleate/Isobornyl Acrylate Copolymer), Aculyne^{®} 258 (Rohm & Haas; Acrylat/ Hydroxyesteracrylat-Copolymer), Luviset^{®} P.U.R. (BASF, Polyurethane-1), Luviflex^{®} Silk (BASF), Eastman^{®} AQ 48 (Eastman), Styleze^{®} CC-10 (ISP; VP/DMAPA Acrylates Copolymer), Styleze^{®} 2000 (ISP; VP/Acrylates/Lauryl Methacrylate Copolymer), DynamX^{®} (National Starch; Polyurethane-14 AMP-Acrylates Copolymer), Resyn^{®} XP (National Starch; Acrylates/Octylacrylamide Copolymer), Fixomer^{®} A-30 (Ondeo Nalco; Polymethacrylsäure (und) Acrylamidomethylpropansulfonsäure), Fixate^{®} G-100 (Noveon; AMP-Acryl ates/Allyl Methacrylate Copolymer).

Geeignete zusätzliche Polymere sind auch die in der US 3,405,084 beschriebenen Terpolymere aus Vinylpyrrolidon, C₁-C₁₀-Alkyl- Cycloalkyl- und Aryl(meth)acrylaten und Acrylsäure. Geeignete zusätzliche Polymere sind weiterhin die in der EP-A-0 257 444 und EP-A-0 480 280 beschriebenen Terpolymere aus Vinylpyrrolidon , tert.-Butyl-(meth)acrylat und (Meth)acrylsäure. Geeignete zusätzliche Polymere sind weiterhin die in der DE-A-42 23 066 beschriebenen Copolymere, die wenigstens einen (Meth)acrylsäureester, (Meth)acrylsäure sowie N-Vinylpyrrolidon und/oder N-Vinylcaprolactam einpolymerisiert enthalten. Auf die Offenbarung dieser Dokumente wird hier Bezug genommen.

Geeignete Carbonsäuregruppen-haltige Polymere sind weiterhin Carbonsäuregruppen-haltige Polyurethane.

Die EP-A-636361 offenbart geeignete Blockcopolymere mit Polysiloxanblöcken und Polyurethan-/Polyharnstoffblöcken, die Carbonsäure- und/oder Sulfonsäuregruppen aufweisen. Geeignete siliconhaltige Polyurethane sind auch in der WO 97/25021 und der EP-A-751 162 beschrieben. Geeignete Polyurethane sind auch in der DE-A-42 25 045 beschrieben, worauf hier in vollem Umfang Bezug genommen wird.

Diese Polyurethane sind prinzipiell aufgebaut aus
i) mindestens einer Verbindung, die zwei oder mehrere aktive Wasserstoffatome pro Molekül enthält,
ii) mindestens einem Carbonsäuregruppen enthaltenden Diol oder einem Salz davon und
iii) mindestens einem Polyisocyanat.

Bei der Komponente i) handelt es sich z. B. um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol% der genannten Verbindungen durch Triole oder Triamine ersetzt sein.

Brauchbare Diole i) sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt. Geeignete Aminoalkohole i) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc. Geeignete Diamine i) sind z. B. Ethylendiamin, Propylendiamin, 1,4-Diaminobutan, 1,5-Diaminopentan und 1,6-Diaminohexan sowie a,w-Diaminopolyether, die durch Aminierung von Polyalkylenoxiden mit Ammoniak herstellbar sind.

Bei der Komponente i) kann es sich auch um ein Polymerisat mit einem zahlenmittleren Molekulargewicht im Bereich von etwa 300 bis 5000, bevorzugt etwa 400 bis 4000, insbesondere 500 bis 3000. Brauchbare Polymerisate i) sind z. B. Polyesterdiole, Polyetherole und Mischungen davon. Polyetherole sind vorzugsweise Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignete Polytetrahydrofurane i) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt. Brauchbare Polyesterdiole i) weisen vorzugsweise ein zahlenmittleres Molekulargewicht im Bereich von etwa 400 bis 5000, bevorzugt 500 bis 3000, insbesondere 600 bis 2000, auf. Als Polyesterdiole i) kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäu ren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, etc.

Geeignete Verbindungen ii), die zwei aktive Wasserstoffatome und mindestens eine Carbonsäuregruppe pro Molekül aufweisen, sind z. B. Dimethylolpropansäure und Mischungen, die Dimethylolpropansäure enthalten.

Bei der Komponente iii) handelt es sich um übliche aliphatische, cycloaliphatische und/oder aromatische Polyisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Geeignete zusätzliche Polymere sind weite rhin kationische Polymere. Dazu zählen z. B. Polymere mit der Bezeichnung Polyquaternium nach INCl, z. B. Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ 11), Copolymere aus N-Vinylcaprolactam/N-Vinylpyrrolidon/N-Vinylimidazoliumsalzen (Luviquat^{®} Hold); kationische Cellulosederivate (Polyquaternium-4 und -10), Acrylamidocopolymere (Polyquaternium-7) und Chitosan. Geeignete kationische (quaternisierte) Polymere sind auch Merquat^{®} (Polymer auf Basis von Dimethyldiallylammoniumchlorid), Gafquat^{®} (quatemäre Polymere, die durch Reaktion von Polyvinylpyrrolidon mit quaternären Ammoniumverbindungen entstehen), Polymer^{®} JR (Hydroxyethylcellulose mit kationischen Gruppen) und kationische Polymere auf pflanzlicher Basis, z. B. Guarpolymere, wie die Jaguar^{®}-Marken der Fa. Rhodia.

Geeignete zusätzliche Polymere sind auch amphotere oder zwitterionische Polymere, wie die unter den Bezeichnungen Amphomer^{®} (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamido propyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette^{®} (AMERCHOL) im Handel erhältlich sind, und Copolymere aus Hydroxyethylmethacrylat, Methylmethacrylat, N,N-Dimethylaminoethylmethacrylat und Acrylsäure (Jordapon^{®}).

Als zusätzliche Polymere geeignete neutrale Polymere sind z. B. Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex^{®} Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol^{®} Plus (BASF), oder Polyvinylpyrrolidon und deren Copolymere, insbesondere mit Vinylestern, wie Vinylacetat, z. B. Luviskol^{®} VA 37 (BASF); Polyamide, z. B. auf Basis von Itaconsäure und aliphatischen Diaminen, wie sie z. B. in der DE-A-43 33 238 beschrieben sind.

Geeignete Polymere sind auch nichtionische, siloxanhaltige, wasserlösliche oder -dispergierbare Polymere, z. B. Polyethersiloxane, wie Tegopren^{®} (Fa. Goldschmidt) oder Belsil^{®} (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien , Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeig neten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere und Polyelektrolytkomplexe geeignet als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

### Pharmazeutische Zubereitungen

Ein Gegenstand der Erfindung betrifft pharmazeutische Mittel enthaltend die erfindungsgemäßen Polymere und pharmazeutisch übliche Wirkstoffe und Zusatzstoffe.

Feste pharmazeutische Darreichungsformen wie Tabletten, Kapseln, Pellets, Granulate, Kristalle etc. werden aus sehr unterschiedlichen Gründen gecoatet, d.h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüber hinaus lässt sich insbesondere die Freisetzungsgeschwindigkeit des Wirkstoffes durch den Filmüberzug einstellen.

Generell unterscheidet man Instant-Release-Formen und Retard- bzw. Slow-Release-Formen.

Bei Instant-Release-Formen sollen der Zerfall der Tablette und die Freisetzung des Wirkstoffs aus der Darreichungsform nach Möglichkeit nicht durch das Coating beeinflußt werden, deshalb muss sich der Filmüberzug schnell im Magensaft auflösen. Daneben muss er über gute Filmeigenschaften verfügen. Die Zugfestigkeit und die Reißdehnung sollten hoch sein, damit der Filmüberzug mechanischen Einwirkungen standhält, wie sie bei der pharmazeutischen Verarbeitung - insbesondere der Konfektionierung - und auch während des Versandes bzw. der Lagerung auftreten.

Ein häufig eingesetztes Produkt für das Coaten von Instant-Release-Tabletten ist Hydroxypropylmethylcellulose (HPMC). Hydroxypropylmethylcellulose weist in wässriger Lösung bei zunehmender Konzentration einen steilen Viskositätsanstieg auf. Ein ähnliches Verhalten zeigt auch Hydroxypropylcellulose (HPC).

Da die Filmbildnerlösung beim Coaten von Tabletten fein zerstäubt werden muss und die gebildeten Tröpfchen die Oberfläche der Tabletten gut benetzen und auch gut spreiten müssen, darf die Viskosität eine gewisse Grenze (zwischen 150 und 250 mPas), die abhängig ist von der Art der Sprühdüse und des Gerätes, nicht überschreiten. Deshalb können im Falle von HPMC nur verhältnismäßig niedrige Filmbildnerkonzentrationen eingesetzt werden.

Als Empfehlung für die Konzentration von Pharmacoat^{®} 606 (Fa. Shin-etsu) werden in der Literatur 5 bis 7 Gew.-% angegeben (Pharmaceutical Coating Technology, Hrsg. Graham Cole, Taylor und Francis Ltd. 1995 und Technische Merkblätter der Hersteller). Diese geringe Sprühkonzentrationen bedingen relativ lange Verarbeitungszeiten und damit hohe Kosten.

Darüber hinaus zeigt Hydroxypropylmethylcellulose weitere Nachteile u.a. im Benetzungsverhalten, im Pigmentbindevermögen, in den mechanischen Eigenschaften der Filme, in der Hygroskopizität sowie in der Permeabilität gegenüber Wasserdampf und Sauerstoff, in der Auflösungsgeschwindigkeit und in der Zerfallszeitdifferenz zwischen Filmtabletten und Kern.

Die geringe Elastizität der Filme aus Hydroxypropylmethylcellulose führt häufig dazu, dass die Filmtabletten bei feuchter Lagerung infolge der Quellung des Kerns aufreißen. Auch der Einsatz von Weichmachern ergibt keine nennenswerten Verbesserungen dieses Problems. Er kann vielmehr zu klebrigen Filmen und durch Migration zu Verä n-derungen der Tabletteneigenschaften führen.

Orale Arzneiformen mit einer Arzneistofffreigabe über einen längeren Zeitraum mit dem Ziel der Wirkungsverlängerung der aktiven Komponente (allgemein Retardarzneiformen) gewinnen zunehmend an Bedeutung. Mit ihr sind eine verbesserte Patientencompliance durch eine reduzierte Einnahmefrequenz, eine Verringerung von Nebenwirkungen durch Vermeidung von Plasmaspitzen, gleichmäßigere Blutspiegel des Arzneistoffs sowie die Vermeidung von lokalen Irritationen vorteilhaft verbunden. Neben der Formulierung von arzneistoffhaltigen Kernen, die mit einem wasserunlöslichen aber semipermeablen bzw. porenhaltigen Film überzogen wurden, durch die der Arzneistoff diffundiert, kann die Steuerung und Verlängerung der Freisetzung durch die Einbettung des Arzneistoffes in Matrices erreicht werden. Weiterhin ist der Einsatz von lonenaustauscherharzen und therapeutischen Systemen (z.B. OROS) möglich. Besonders die Einbettung des Arzneistoffs in Hydrokolloidmatrices bietet die Vorteile einer einfachen und preiswerten Herstellung und einer hohen Arzneimittelsicherheit, da Dose Dumping Effekte nicht auftreten können. Die hierfür in der Regel eingesetzten Hilfsstoffe wie Hydroxypropylmethylcellulose (HPMC), Hydroxypropylcellulose, Alginsäure bzw. Alginate sowie Xanthan besitzen Anwendungsnachteile. Hier sind zu nennen: Mangelnde Fließeigenschaften, die eine Direkttablettierung erschweren, eine Abhängigkeit der Arzneistofffreigabe von der Osmolarität (Salzgehalt) und vom pH-Wert des Freisetzungsmediums. Dies gilt ebenso für HPMC wie für Hydroxypropylcellulose, Xanthan und Alginate. Die Verwendung von Xanthan führt ferner zu Tabletten mit geringer Härte, die Direkttablettierung von Alginaten resultiert in Presslingen mit nur noch geringen retardierenden Eigenschaften (max. 8 h). Die natürlichen Quellstoffe (z.B. Alginate) besitzen insgesamt eine starke Chargenvariabilität.

Bindemittel werden in pharmazeutischen Darreichungsformen eingesetzt, um die Verarbeitbarkeit und die mechanische Festigkeit zu erhöhen. Sie werden üblicherweise in Tabletten, Granulaten und Pellets eingesetzt und führen zu verbesserter Fließfähigkeit, höherer Bruchfestigkeit und geringerer Friabi lität.

Die derzeit verwendeten Bindemittel wie Maltodextrin oder Polyvinylpyrrolidone führen häufig zu nicht zufriedenstellenden Bruchfestigkeiten und Friabilitäten. Andere Bindemittel wie Stärkekleister und Hydroxypropylmethylcellulose (HPMC) lassen sich aufgrund ihrer hohen Viskosität nur niedrigkonzentriert einsetzen.

Weiterhin werden filmbildende Hilfsstoffe in Lösungen und Sprays eingesetzt, die auf der Haut oder Schleimhaut aufgebracht oder auch dem Körper systemisch zugeführt werden. Beispiele hierfür sind Zubereitungen für die Wundbehandlung, Sprayverbände aber auch Zubereitungen zur Applikation auf intakter Haut bzw. Schleimhaut. Dabei wird die Haut durch einen Film geschützt und die Wirkstoffe können in bzw. durch die Haut dringen.

Bei transdermalen therapeutischen Systemen und bei Wundpflastern ist ebenfalls wie bei den oben genannten Darreichungsformen eine ausreichende Flexibilität erforderlich, die die derzeit zur Verfügung stehenden Produkte nicht aufweisen. Der Einsatz von möglichen Weichmachern zur Erreichung der notwendigen Flexibilität ist aus toxikologischen und pharmakologischen Gründen nicht erwünscht.

Eine Aufgabe der vorliegenden Erfindung bestand darin, wasserlösliche oder wasserdispergierbare Polymerisate als Überzugsmittel, Bindemittel und/oder Filmbildner in pharmazeutischen Zubereitungen bereitzustellen, die die oben genannten Nachteile nicht aufweisen.

Überraschenderweise wurde gefunden, dass sich die erfindungsgemäßen Polymere zur Verwendung in pharmazeutischen Zubereitungen eignen.

Sie eignen sich insbesondere als Überzugsmittel, Bindemittel, Filmbildner und auch als Matrix für die Wirkstofffreisetzung in pharmazeutischen Zubereitungen.

Die erfindungsgemäßen Polymere können in einer Vielzahl von pharmazeutischen Zubereitungen eingesetzt werden.

Beispielsweise können genannt werden als überzogene Zubereitungen Filmtabletten, Filmmikrotabletten, Dragees, überzogene Pastillen, Kapseln, Kristalle, Granulate oder Pellets.

Bei bindemittelhaltige Zubereitungen handelt es sich um beispielsweise Tabletten, Mikrotabletten, Kerne, Granulate oder Pellets.

Weiterhin können die erfindungsgemäßen Polymere zur Herstellung von Lösungen und Sprays verwendet werden, die, auf Haut oder Schleimhaut aufgebracht, einen Film ausbilden.

Beispiele hierfür sind Sprühverbände für Wunden, Desinfektionssprays, Lösungen mit Mycostatica, Mundsprays oder-lösungen mit Antibiotika etc.. Auch der Einsatz bei transdermalen therapeutischen Systemen möglich.

Die erfindungsgemäßen Polymere benetzen leicht lipophile Oberflächen und besitzen hervorragende Schutzkolloideigenschaften. Eingearbeitet in Suspensionen und Emulsionen lagern sie sich an die Teilchen der dispersen Phase und stabilisieren diese. Sie können daher als Benetzungshilfsmittel und Stabilisatoren in dispersen Systemen verwendet werden.

Durch Wechselwirkung mit schwer wasserlöslichen Arzneistoffen verbessern sie deren Löslichkeit und Lösungsgeschwindigkeit, wodurch Resorbierbarkeit und Bioverfügbarkeit der Arzneistoffe verbessert werden. Diese vorteilhafte Wirkung zeigt sich beispielsweise bei den Darreichungsformen, bei denen der Wirkstoff nicht gelöst vorliegt, wie z.B. Tabletten, Granulate, Suspensionen etc..

Die die erfindungsgemäßen Polymere können gegebenenfalls auch in Kombi nation mit anderen Hilfsstoffen zusammen mit Wirkstoffen zu Polymer-Wirkstoffschmelzen verarbeitet werden, die entweder zu Arzneistoffen extrudiert und kalandriert werden oder nach der Extrusion zu Granulaten oder Pulvern zerteilt werden und erst anschließend in Arzneiformen verarbeitet werden, beispielsweise zu Tabletten verpresst werden.

Dabei bringen die erfindungsgemäßen Polymere die bereits oben aufgeführten Eigenschaften in die Zubereitungen ein.

In verschiedenen pharmazeutischen Zubereitungen können die erfindungsgemäßen Polymere folgende Funktionen hervorragend erfüllen:

Dispergierhilfsstoff, Suspendierhilfsstoff, Benetzungsmittel, Solubilisator für schwerlösliche Arzneistoffe, Emulgator, Kristallisationsinhibitor, Anticakinghilfsstoff, Schutzkolloid, Bioadhäsivum zur Verlängerung und Intensivierung des Kontaktes mit der Schleimhaut, Spreithilfsmittel, Viskositätsregulator, Hilfsstoff zur Herstellung von festen Lösungen mit Arzneistoffen, Hilfsstoff zur Ei nstellung der Wirkstofffreisetzung in Retardformulierungen.

Die Löslichkeit der erfindungsgemäßen Polymere kann durch geeignete Wahl des Neutralisationsgrades in gewissen Grenzen eingestellt werden.

So können in Wasser nicht oder nur gering lösliche, aber dispergierbare erfindungsgemäße Polymere auch als Retardierungspolymere verwendet werden.

Bei der Verwendung zur Herstellung von Suppositorien und Vaginalglobuli gewährleisten die die erfindungsgemäßen Polymere ei nerseits die Flexibilität der Darreichungsform und fördern andererseits den Zerfall und die Wirkstoffauflösung und sie kleiden die Schleimhaut mit einem wirkstoffhaltigen Film aus, der die Resorption verstärkt.

Tabletten quellen in Abhängigkeit von den verwendeten Hilfs- und Wirkstoffen, der Lagerzeit und den Lagerbedingungen, wie Temperatur und Feuchtigkeit, unterschiedlich stark. Ein starrer Filmüberzug erleidet bei Quellung des Kernes Risse. Deshalb ist die Elastizität von Filmbildnern eine wichtige Größe.

Die gegebenenfalls neutralisierten erfindungsgemäßen Polymere können in reiner Form oder aber zusammen mit den üblichen Hilfsstoffen auf den wirkstoffhaltigen Kern appliziert werden. Übliche Hilfsstoffe sind z. B. Farbpigmente zur Einfärbung, Weißpigmente, wie Titandioxid, zur Erhöhung der Deckkraft, Talkum und Siliciumdioxid als Antiklebemittel, Polyethylenglykole, Glycerin, Propylenglykol, Triacetin, Triethylcitrat als Weichmacher und verschiedene oberflächenaktive Stoffe, wie Natriumlaurylsulfat, Polysorbat 80, Pluronics und Cremophore, zur Verbesserung des Benetzungsverhaltens. Die exemplarisch genannten Stoffe stellen keine Begrenzung dar. Es können alle bekanntermaßen für magensaftlösliche Filmüberzüge geeigneten Zusatzstoffe verwendet werden.

Als Überzugsverfahren lassen sich die gebräuchlichen Verfahren, wie das Coaten in der Wirbelschicht oder im Horizontaltrommelcoater, das Tauchschwertverfahren und das Kesselcoatingverfahren, anwenden. Neben der Anwendung auf Tabletten können die erfindungsgemäßen Polymere auch für das Coating von anderen pharmazeutischen Zubereitungen, wie Granulaten, Pellets, Kristallen oder Kapseln, eingesetzt werden. Die neuen Überzugsmittel werden wie üblich in einer Stärke von 5 bis 200 µm, vorzugsweise 10 bis 100 µm, aufgetragen.

Bei der Verwendung als Bindemittel unterscheidet man je nach Verarbeitungsverfahren zwischen Feucht- und Trockenbindemittel. Letztere werden u.a. bei der Direkttablettierung und bei der Trockengranulation bzw. Kompaktierung verwendet. Hierbei wird das Bindemittel mit dem Wirkstoff und gegebenenfalls weiteren Hilfsstoffen vermischt und anschließend direkttablettiert oder granuliert bzw. kompaktiert.

Im Gegensatz dazu wird bei der Feuchtgranulation die Wirkstoff-Hilfsstoffmischung mit einer Lösung des Bindemittels in Wasser oder einem organischen Lösungsmittel befeuchtet, die feuchte Masse durch ein Sieb gegeben und anschließend getrocknet. Befeuchtung und Trocknung können dabei auch parallel ablaufen, wie z.B. in der Wirbelschichtgranulation.

Für eine optimale Verarbeitung soll das Bindemittel in Lösung niedrigviskos sein, da viskose Lösungen zu inhomogenen Granulaten führen.

Ein Bindemittel soll zu gleichmäßigen, harten, abriebsstabilen Granulaten bzw. Tabletten führen. Insbesondere bei Tabletten kommt der Bruchfestigkeit besondere Bedeutung zu, da sich viele Wirkstoffe schlecht verpressen lassen und somit Tabletten mit unzureichender mechanischer Stabilität ergeben.

Weiterhin soll der Zerfall der Arzneiformen sowie die Freisetzungsgeschwindigkeit der Wirkstoffe durch das Bindemittel nicht nennenswert nachteilig beeinflusst werden.

Die gängigsten Bindemittel sind beispielsweise Polyvinylpyrrolidon, Vinylacetat-Vinylpyrrolidon Copolymere, Gelatine, Stärkekleister, Maltodextrine, hydroxyalkylierte bzw. carboxyalkylierte Cellulosederivate, wie Hydroxypropylmethylcellulose, Methylcellulose, Natriumcarboxymethylcellulose sowie natürliche Gummisorten, wie beispielsweise Gummi Arabicum, Pektin oder Alginat.

Viele dieser Bindemittel weisen in Lösung eine hohe Viskosität auf und sind schlecht verarbeitbar. Durch die hohe Viskosität werden die zu granulierenden Pulverteilchen schlecht und ungleichmäßig benetzt, so dass daraus eine zu geringe Granulatfestigkeit und eine ungünstige Korngrößenverteilung resultieren.

Viele Bindemittel sind zudem hygroskopisch und quellen bei Wasseraufnahme. Dadurch können sich die Granulat- und Tabletteneigenschaften dramatisch verändern.

Überraschenderweise wurde nun gefunden, dass die sulfonierten, Sulfongruppen enthaltenden Polymere über ausgezeichnete Bindemittelwirkungen verfügen und zudem in Konzentrationsbereichen von 0,5 bis 20 Gew.-%, bevorzugt 1 bis 10 Gew.-% der Gesamtmenge der Formulierung den Zerfall nicht nennenswert beeinflussen. Aufgrund des guten Benetzungsverhaltens kann sich zudem die Freisetzung von schlecht löslichen Wirkstoffen verbessern. Hervorzuheben ist auch die vergleichsweise geringe Viskosität der Polymerlösungen.

Bei Bedarf können selbstverständlich die pharmazeutisch akzeptablen, für haut- und/oder haarkosmetische Zubereitungen geeigneten Zusatzstoffe und Hilfsmittel in den pharmazeutischen Zubereitungen verwendet werden.

Mit den Polymerisaten als Bindemittel ergeben sich mechanisch außerordentlich stabile und auch über lange Lagerzeiten stabile Granulate bzw. Tabletten.

Eine weitere bevorzugte Ausführungsform der Erfindung sind Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Eine weitere bevorzugte Ausführungsform der Erfindung sind kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deod o-rantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die erfindungsgemäßen Copolymere in Nose-Strips zur Porenreinigung, in Antiaknemitteln, Repellents, Rasiermitteln, Haarentfernungsmitteln, Intimpflegemitteln, Fußpflegemitteln sowie in der Babypflege verwendet werden.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der erfindungsgemäßen Copolymere zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein erfindungsgemäßes Copolymer in ei nem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der erfindungsgemäßen Copolymere besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den erfindungsgemäßen Copolymere und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Copolymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem erfindungsgemäßen Copolymer in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein erfindungsgemäßes Copolymer eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole, enthalten.

Neben den erfindungsgemäßen Copolymeren können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein erfindungsgemäßes Copolymer sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesam t-gewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocam phopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propyleneglykol-Oleat, PEG-120-Methylglucosedioleat und andere, sowie Konservierungsmittel, weitere Wirk- und Hilfsstoffe und Wasser enthalten.

Eine besonders bevorzugte Ausführungsform der Erfindung sind Haarbehandlungsrnittel.

Erfindungsgemäße Haarbehandlungsmittel enthalten vorzugsweise wenigstens ein erfindungsgemäßes Copolymer in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetische n Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

In einer bevorzugten Ausführungsform der Erfindung enthalten die erfindungsgemäßen Mittel einen Anteil an flüchtigen organischen Komponenten (VOC) von höchstens 80 Gew.-%, besonders bevorzugt höchstens 55 Gew.-%.

Die erfindungsgemäßen haarkosmetischen Formulierungen enthalten in einer bevorzugten Ausführungsform
a) 0,05 bis 20 Gew.-% wenigstens eines erfindungsgemäßen Copolymers,
b) 20 bis 99,95 Gew.-% Wasser und/oder Alkohol,
c) 0 bis 50 Gew.-% wenig stens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaum bildner und Solubilisatoren. Die eingesetzten grenzflächenaktiven Verbindungen können anionisch, kationisch, amphoter oder neutral sei n. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

Alle für kosmetische Mittel geeigneten Inhaltsstoffe können gegebenenfalls auch für die haarkosmetischen Mittel verwendet werden.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Copolymere eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Spray-Zubereitungen
a) 0,1 bis 10 Gew.-% wenigstens eines erfindungsgemäßen Copolymers,
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 bis 10 Gew.-% wenigstens eines erfindungsgemäßen Copolymers,
b) 55 bis 99,8 Gew.-% Wasser und/oder Alkohol,
c) 5 bis 20 Gew.-% eines Treibmittel,
d) 0,1 bis 5 Gew.-% eines Emulgators,
e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Für Aerosolschäume besonders geeignete Treibmittel sind Mischungen aus Dimethylether und, gegebenenfalls halogenierten, Kohlenwasserstoffen wie Propan, Butan, Pentan oder HFC-152 a.

Beispiele für nichtionische Emulgatoren (INCl-Nomenklatur) sind Laurethe, z- B. Laureth-4; Cetethe, z. B. Cetheth-1, Polyethylenglycolcetylether; Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylam moniumdihydrogenphosphat, Cetyltrimoniumchlorid, Cetyltrimmoniumbromid, Cocotrimoniummethylsulfat, Quaternium-1 bis x (INCl).

Anionische Emulgatoren können beispielsweise ausgewählt werden aus de r Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkyl phosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 10 Gew.-% wenigstens eines erfindungsgemäßen Copolymers,
b) 80 bis 99,85 Gew.-% Wasser und/oder Alkohol,
c) 0 bis 3 Gew.-%, bevorzugt 0,05 bis 2 Gew.-%, eines Gelbildners,
d) 0 bis 20 Gew.-% weitere Bestandteile.

Der Einsatz von Gelbildnern kann jedoch von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCl), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCl), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Die erfindungsgemäßen Copolymere können in kosmetischen Zubereitungen als Konditioniermittel eingesetzt werden.

Die erfindungsgemäßen Copolymere, wie zuvor definiert, können bevorzugt in Shampooformulierungen als Festigungs- und/oder Konditioniermittel eingesetzt werden. Bevorzugte Shampooformulierungen enthalten
a) 0,05 bis 10 Gew.-% wenigstens eines erfindungsgemäßen Copolymers,
b) 25 bis 94,95 Gew.-% Wasser,
c) 5 bis 50 Gew.-% Tenside,
c) 0 bis 5 Gew.-% eines weiteren Konditioniermittels,
d) 0 bis 10 Gew.-% weitere kosmetische Bestandteile.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlau rylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Copolymeren eingesetzt werden. Hierzu zählen beispielsweise die zuvor genannten kationischen Polymere mit der Bezeichnung Polyquaternium nach INCl, insbesondere Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ 11), Copolymere aus N-Vinylcaprolactam/N-Vinylpyrrolidon/N-Vinylimidazoliumsalzen (Luviquat^{®} Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCl) verwendet werden.

Ein weiterer Gegenstand der Erfindung ist die Verwendung eines Copolymers, wie zuvor definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden nicht einschränkenden Beispiele näher erläutert.

**Herstellung der Copolymere:**

| Herstellungsvorschrift für Copolymere, die kein N-Vinyllactam enthalten | | |
|---|---|---|
| (Verfahren A): | | |
| Beispiel Nr. 10 | 500g 30Gew.-%ige Ethanol-Wasser [2:1]- Lösung von P (EMA/MAS/AS/NtBAEMA) 72:22: 3 :3 | |
| | | |
| Vorlage | 102g | Wasser |
| | 63g | Ethanol |
| | 13.1 g | Zulauf 1 |
| | 0.81g | Zulauf 2 |
| | | |
| Zulauf 1: | 108.0g | Ethylmethacrylat |
| | 33.0g | Methacrylsäure |
| | 4.5g | Acrylsäure |
| | 4.5g | N-tert.Butylaminoethylmethacrylat |
| | 1 12.0 g | Ethanol |
| | | |
| Zulauf 2 | 2.25g | Waco V50 |
| | 14 g | Wasser |
| | | |
| Zulauf 3 | 0.75 | tert.- Butylperpivalat 75%ig |
| | 29.75g | Ethanol |
| | | |
| Zulauf 4 | 39.7g | Aminomethylpropanol AMP |
| | 26.48 | Wasser |
| | 96g | Ethanol |

In einer Rührapparatur mit Rückflusskühler, Innenthermometer und vier separaten Zulaufvorrichtungen wurden 13.1 g von Zulauf 1, 0.81 von Zulauf 2, 102.0 g Wasser und 63 g Ethanol vorgelegt und die Mischung unter Rühren auf ca. 75°C aufgeheizt.

Nach dem Anpolymerisieren, erkennbar an einer leichten Viskositätserhöhung, wurde bei 75°C der Rest von Zulauf 1 innerhalb von drei Stunden und der Rest von Zulauf 2 innerhalb von vier Stunden zugegeben. Die Reaktionslösung wurde noch ca. zwei Stunden bei 75°C nachgerührt. Der Zulauf 3 wurde bei ca. 75°C in 30 Minuten zudosiert. Die Polymermischung wurde noch ca. zwei Stunden bei 80°C nachpolymerisiert. Die Polymerlösung wurde mit AMP in Zulauf 4 in 10 Minuten neutralisiert. Man erhält 500g einer 30 %- wässrig ethanolischen Polymerlösung.

Nach dieser Vorschrift wurden die Produkte 1 -13 (Tab.1) synthetisiert.

| Herstellungsvorschrift für Copolymere, die N-Vinyllactam enthalten (Verfahren B): | | |
|---|---|---|
| Beispiel Nr. 19 | 500g 30%ige Ethanol-Wasser [30:40]- Lösung von | |
| | P(EMA/VP/MAS/VI) 50:30:15:5 | |
| | | |
| Vorlage | 64.0g | Wasser |
| | 174g | Ethanol |
| | 8.12 g | Zulauf 1 |
| | 2.78 g | Zulauf 2 |
| | | |
| Zulauf 1: | 75g | Ethylmethacrylat |
| | 45.0g | Vinylpyrrolidon |
| | 22.5g | Methacrylsäure |
| | 7.5g | Vinylimidazol |
| | 12.42 | N,N-Dimethylethanolamin |
| | | |
| Zulauf 2 | 3.0 g | Waco^{®}V50 |
| | 52.5 g | Wasser |
| | | |
| Zulauf 3 | 0.75 | tert.- Butylperpivalat 75%ig |
| | 29.75g | Ethanol |

In einer Rührapparatur mit Rückflusskühler, Innenthermometer und vier separaten Zulaufvorrichtungen wurden 8,12 g von Zulauf 1, 2,78 g von Zulauf 2, 64g Wasser und 174g Ethanol vorgelegt und die Mischung unter Rühren auf ca. 65°C aufgeheizt.

Nach dem Anpolymerisieren, erkennbar an einer leichten Viskositätserhöhung, wurde bei 67°C der Rest von Zulauf 1 innerhalb von drei Stunden und der Rest von Zulauf 2 innerhalb von vier Stunden zugegeben. Die Reaktionslösung wurde noch ca. zwei Stunden bei 70°C nachgerührt.
Der Zulauf 3 wurde bei ca. 70°C in 30 Minuten zudosiert. Die Polymermischung wurde noch ca. zwei Stunden bei 80°C nachpolymerisiert. Anschließend bei einer Außentemperatur Ta von 120°C wurde die Reaktionsmischung durch Wasserdampf destilliert bis eine Innentemperatur Ti von ca. 100°C erreichte. Die Polymerlösung wurde weiter mit Wasserdampf noch 1 Stunde bei 100°C behandelt, dann mit Aminomethylp ropanol auf einem pH 8 - 8.4 eingestellt. Die viskose, trübe Polymerlösung wurde mit 150g Ethanol stabilisiert und mit Wasser auf einem Feststoffgehalt von 30% verdünnt. Man erhält danach 500g einer 30 % leicht trüben, stabilen Polymerlösung.

Nach dieser Vorschrift wurden die Produkte 14-22 (Tab.2) synthetisiert.

### Verwendete Abkürzungen:

- MAS: Methacrylsäure
- AS: Acrylsäure
- VCap: Vinylcaprolactam
- VP: N-Vinylpyrrolidon
- MMA: Methylmethacrylat
- EMA: Ethylmethacrylat
- n-BMA: n-Butylmethacrylat
- t-BMA: t-Butylmethacrylat
- t-BA: t-Butylacrylat
- EA: Ethylacrylat
- EHA: Ethylhexylacrylat
- n-BA: n-Butylacrylat
- HEMA: Hydroxyethylmethacrylat
- HEA: Hydroxyethylacrylat
- HPA: Hydroxypropylacrylat
- NtBAEMA: N-tert.Butylaminoethylmethacrylat
- VI: Vinylimidazol
- DMAPMA: N-[3-(dimethylamino)propyl]methacrylamid
- w/w: im Gewichtsverhältnis
- WS: Wirkstoff
- DME: Dimethylether

**Tab. 1 Erfindungsgemäße Copolymere ohne N-Vinyllactame (Komponente b))**

| Bsp. | EMA | t-BA | VP | VCa p | MAS | AS | NtBA EMA | VI | DMA PMA | Verfahren |
|---|---|---|---|---|---|---|---|---|---|---|
| 1 | 75 | -- | -- | -- | 24 | -- | -- | -- | 1 | A |
| 2 | 75 | -- | -- | -- | 20 | 3 | -- | -- | 2 | A |
| 3 | 70 | -- | -- | -- | 24 | 3 | -- | -- | 3 | A |
| 4 | 73 | -- | -- | -- | 25 | -- | 2 | -- | -- | A |
| 5 | 72 | -- | -- | -- | 22 | 3 | 3 | -- | -- | A |
| 6 | 72 | -- | -- | -- | 20 | 5 | 3 | -- | -- | A |
| 7 | 70 | -- | -- | -- | 24 | 3 | 3 | -- | -- | A |
| 8 | 78 | -- | -- | -- | 20 | -- | -- | 2 | -- | A |
| 9 | 75 | -- | -- | -- | 23 | 2 | -- | 5 | -- | A |
| 10 | 72 | -- | -- | -- | 22 | 3 | -- | 3 | -- | A |
| 11 | 65 | -- | -- | -- | 25 | 5 | -- | 5 | -- | A |
| 12 | 55 | -- | -- | -- | 25 | 10 | -- | 10 | -- | A |
| 13 | 50 | 15 | -- | -- | 25 | 5 | -- | 5 | -- | A |

Die gelösten erfindungsgemäßen Copolymere ohne N-Vinyllactame der Tab.1 wurden mit AMP (2-Amino-2-Methyl-propanol) zu 100% neutralisiert.

**Tab.2 Erfindungsgemäße Copolymere enthaltend N-Vinyllactamen (Komponente b))**

| Bsp. | EMA | t-BA | VP | VCa p | MAS | AS | NtBA EMA | VI | DMA PMA | Verfahren |
|---|---|---|---|---|---|---|---|---|---|---|
| 14 | 65 | -- | 10 | -- | 23 | -- | -- | 2 | -- | B |
| 15 | 58 | -- | 20 | - | 18 | -- | 4 | -- | -- | B |
| 16 | 58 | -- | 20 | -- | 18 | -- | -- | 4 | -- | B |
| 17 | 58 | -- | 20 | -- | 18 | -- | -- | -- | 4 | B |
| 18 | 30 | 18 | 20 | -- | 18 | - | -- | -- | 4 | B |
| 19 | 50 | -- | 30 | -- | 15 | -- | -- | 5 | -- | B |
| 20 | 50 | -- | 30 | -- | 13 | -- | -- | 7 | -- | B |
| 21 | 50 | -- | -- | 25 | 20 | -- | -- | 5 | -- | B |
| 22 | 40 | -- | -- | 35 | 20 | -- | 5 | -- | -- | B |
| 23* | 50 | -- | 30 | | 10 | | | 10 | -- | B |

| | | | | | | | | | | |
|---|---|---|---|---|---|---|---|---|---|---|
| *nicht erfindungsgemäß | | | | | | | | | | |

Der pH-Wert der Polymerisationslösungen der erfindungsgemäßen Copolymere mit N-Vinyllactamen der Tab.2 wurden mit AMP (2-Amino-2-Methyl-propanol) auf einen pH-Wert im Bereich von 8,0 bis 8,4 eingestellt.

### Messung der Festigung (Biegesteifigkeit):

Die Festigung von polymeren Filmbildnern wurde außer durch subjektive Beurteilung auch objektiv physikalisch als Biegesteifigkeit von dünnen Haarsträhnen gemessen, die mit der Polymerlösung behandelt und wieder getrocknet wurden. Dabei bestimmt ein Kraftaufnehmer die zum Biegen erforderliche Kraft, während die gesamte Messung unter standardisierten Bedingungen in einem Klimaraum bei 65 % relativer Luftfeuchte abläuft.

Zur Messung der Biegesteifigkeit wurden 3,0 gew.-%ige Lösungen der erfindungsgemäßen Polymerisate hergestellt. Die Messung der Biegesteifigkeit wurde an 5 bis 10 Haarsträhnen (ä ca. 3 g und 24 cm Länge) bei 20°C und 65 % relativer Feuchte durchgeführt. Die gewogenen, trockenen Haarsträhnen wurden in die 3,0 gew.%ige Polymerlösung getaucht, wobei durch dreimaliges Eintauchen und Herausnehmen eine gleichmäßige Verteilung sichergestellt wurde. Die überschüssige Filmbildne rlösung wurde dann zwischen Daumen und Zeigefinger abgestreift und die Haarsträhnen anschließend durch Ausdrücken zwischen Filterpapier sorgfältig ausgedrückt. Danach wurden die Strähnen von Hand so geformt, dass sie einen runden Querschnitt erhielten. Bei 20°C und 65 % relativer Feuchte wurde über Nacht im Klimara um getrocknet.

Die Prüfungen wurden in einem Klimaraum bei 20°C und 65 % relativer Feuchte mittels eines Zug/Druck-Prüfgerätes durchgeführt. Die Haarsträhne wurde symmetrisch auf zwei zylindrische Rollen der Probenaufnahme gelegt. Genau in der Mitte wurde die Strähne nun von oben mit einem abgerundetem Stempel 40 mm durchgebogen (Brechen des Polymerfilms). Die dafür erforderliche Kraft wurde mit einer Wägezelle (50 N) gemessen und in Newton angegeben.

### 1) Bestimmung des K-Wertes

Die K-Werte werden nach Fikentscher, Cellulosechemie, Bd. 13, S. 58 bis 64 (1932) bei 25°C in wässrig/ethanolischer oder ethanolischer Lösung gemessen und stellen ein Maß für das Molgewicht dar. Die wässrig/ethanolische oder ethanolische Lösung der Polymerisate enthält 1g Polymerisat in 100 ml Lösung. Für den Fa II, dass die Polymerisate in Form von wässrigen Dispersionen vorliegen, werden in Abhängigkeit vom Polymergehalt der Dispersion entsprechende Mengen der Dispersion mit Ethanol auf 100 ml aufgefüllt, so dass die Konzentration von 1 g Polymerisat in 100 ml Lösung entstehen.

Die Messung des K-Wertes erfolgt in einer Micro-Ubbelohde-Kapillare Typ M Ic der Fa. Schott.

| Beispiel | EMA | MMA | VP | MAS | VI | DMAP MAM | K-Wert | Biegesteifigkeit [cN] |
|---|---|---|---|---|---|---|---|---|
| 23 | 50 | - | 30 | 10 | 10 | -- | 39,5 | 380 cN |

### Anwendungstechnische Beispiele

### Beispiel A1:

| VOC 80-Aerosol-Haarspray | Gew.-% |
|---|---|
| | |
| Polymer aus Beispiel Nr. 1 | 12,00 |
| (30 %ige wässrig-ethanolische Dispersion) | |
| Wasser | 13,60 |
| Dimethylether | 40,00 |
| Ethanol | 34,40 |
| weiterer Zusatz: Silikon, Parfüm, Entschäumer ... | |

Das Beispiel lässt sich mit den Polymeren der Beispiele 2-13 wiederholen. Es wird jeweils ein VOC 80-Aerosol-Haarspray mit guten Eigenschaften erhalten.

### Beispiel A1a):

| VOC-80-Aerosol-H aarspray | Gew.-% |
|---|---|
| | |
| Polymer aus Beispiel Nr. 14 | 12,00 |
| (30 %ige wässrig-ethanolische Dispersion) | |
| Wasser | 11,60 |
| Dimethylether | 40,00 |
| Ethanol | 36,40 |
| weiterer Zusatz: Silikon, Parfüm, Entschäumer ... | |

Das Beispiel lässt sich mit den Polymeren der Beispiele 15-22 wiederholen. Es wird jeweils ein VOC 8O-Aerosol-Haarspray mit guten Eigenschaften erhalten.

### Beispiel A2:

| VOC 80-Aerosol-H aarspray | Gew.-% |
|---|---|
| | |
| Polymer aus Beispiel Nr. 1 | 12,00 |
| (30 %ige wässrig-ethanolische Dispersion) | |
| Ultrahold^{®}Strong | 1,0 |
| Wasser | 12,20 |
| Dimethylether | 40,00 |
| Ethanol | 34,40 |
| Aminomethylpropanol (95%ig) | 0,40 |
| weiterer Zusatz: Silikon, Parfüm, Entschäumer ... | |

Das Beispiel lässt sich mit den Polymeren der Beispiele 2 bis 13 wiederholen. Es wird jeweils ein VOC 8O-Aerosol-Haarspray mit guten Eigenschaften erhalten.

### Beispiel A3:

| VOC 80-Aerosol-Haarspray | Gew.-% |
|---|---|
| | |
| Polymer aus Beispiel Nr. 1 | 12,00 |
| (30 %ige wässrig-ethanolische Dispersion) | |
| Luviset P.U.R.. (30 %ige wässrig-ethanolische Lösung) | 5,0 |
| Wasser | 07,10 |
| Dimethylether | 40,00 |
| Ethanol | 35,90 |
| Aminomethyl propanol (95%ig) weiterer Zusatz: Silikon, Parfüm, Entschäumer ... | bis pH 8.3 |

Das Beispiel lässt sich mit den Polymeren der Beispiele 2 bis 22 wiederholen. Es wird jeweils ein VOC 80-Aerosol-Haarspray mit guten Eigenschaften erhalten.

### Beispiel A4:

| VOC 55 Aerosol-Haarspray | Gew.-% |
|---|---|
| | |
| Polymer aus Beispiel Nr. 1 | 15,00 |
| (30 %ige wässrig-ethanolische Dispersion) | |
| Wasser | 37,00 |
| Dimethylether | 40,00 |
| Ethanol | 8,00 |
| weiterer Zusatz: Silikon, Parfüm, Entschäumer ... | |

Das Beispiel lässt sich mit den Polymeren der Beispiele 2 bis 20 wiederholen. Es wird jeweils ein VOC 55-Aerosol-Haarspray mit guten Eigenschaften erhalten.

### Beispiel A5:

| VOC 55 Aerosol-Haarspray | Gew.-% |
|---|---|
| | |
| Polymer aus Beispiel Nr. 1 | 12,00 |
| (30 %ige wässrig-ethanolische Dispersion) | |
| Luviset^{®}P.U. R. (30% ige wässrig-ethanolische Dispersion) | 3,00 |
| Wasser | 35,90 |
| Dimethylether | 40,00 |
| Ethanol | 9,10 |
| weiterer Zusatz: Silikon, Parfüm, Entschäumer ... | |

Das Beispiel lässt sich mit den Polymeren der Beispiele 2 bis 20 wiederholen. Es wird jeweils ein VOC 55-Aerosol-Haarspray mit guten Eigenschaften erhalten.

### Beispiel A6:

| VOC 55 Aerosol-Haarspray | Gew.-% |
|---|---|
| | |
| Polymer aus Beispiel Nr. 1 | 12,00 |
| (30 %ige wässrig-ethanolische Dispersion) | |
| Luvimer^{®}Pro55 ( 30%ige Dispersion) | 3,00 |
| Wasser | 35,90 |
| Dimethylether | 40,00 |
| Ethanol | 8,80 |
| Aminomethylpropanol (95%ig) | 0,30 |
| weiterer Zusatz: Silikon, Parfüm, Entschäumer ... | |

Das Beispiel lässt sich mit den Polymeren der Beispiele 2 bis 20 wiederholen. Es wird jeweils ein VOC 55-Aerosol-Haarspray mit guten Eigenschaften erhalten.

### Beispiel A7:

| VOC 55 Aerosol-Haarspray | Gew.-% |
|---|---|
| | |
| Polymer aus Beispiel Nr. 1 | 12,00 |
| (30 %ige wässrig-ethanolische Dispersion) | |
| Stepanhold^{®} R-1 *) (Fa. Stepan Chemical Co.) | 1,00 |
| Wasser | 37,40 |
| Dimethylether | 40,00 |
| Ethanol | 9,40 |
| Aminomethylpropanol (95%ig) | 0,20 |
| weiterer Zusatz: Silikon, Parfüm, Entschäumer ... | |

| | |
|---|---|
| *) Stepanhold R-1 = Poly(Vinylpyrrolidon/Ethylmethacrylat/Methacrylsäure) | |

Das Beispiel lässt sich mit den Polymeren der Beispiele 2 bis 20 wiederholen. Es wird jeweils ein VOC 55-Aerosol-Haarspray mit guten Eigenschaften erhalten.

### Beispiel A8:

| VOC 55 Pumpspray | Gew.-% |
|---|---|
| | |
| Polymer aus Beispiel Nr. 1 | 15,00 |
| (30 %ige wässrig-ethanolische Dispersion) | |
| Wasser | 37,00 |
| Ethanol | 48,00 |
| weiterer Zusatz: Silikon, Parfüm, Entschäumer ... | |

Das Beispiel lässt sich mit den Polymeren der Beispiele 2 bis 20 wiederholen. Es wird jeweils ein VOC 55-Pumpspray mit guten Eigenschaften erhalte n.

### Beispiel A9:

| VOC 55 Pumpspray | Gew.-% |
|---|---|
| Polymer aus Beispiel Nr. 1 | 12,00 |
| (30 %ige wässrig-ethanolische Dispersion) | |
| Luviset^{®} Clear ^{*)} (BASF AG) 20%ig | 5,00 |
| Wasser | 33,60 |
| Ethanol | 49,40 |
| weiterer Zusatz: Silikon, Parfüm, Entschäumer ... | |

| | |
|---|---|
| ^{*)} Luviset^{®} Clear = Poly(Vinylpyrrolidon/Methcarylamid/Vinylimidazol) | |

Das Beispiel lässt sich mit den Polymeren der Beispiele 2 bis 20 wiederholen. Es wird jeweils ein VOC 55-Pumpspray mit guten Eigenschaften erhalten.

### Beispiel A10:

| VOC <10- Pumpspray | Gew. -% |
|---|---|
| | |
| Polymer aus Beispiel Nr. 1 | 12,00 |
| (30 %ige wässrig-ethanolische Dispersion) | |
| Luviset^{®} Clear*⁾ (BASF AG) 20%ig | 5,00 |
| Wasser | 83,00 |
| weiterer Zusatz: Silikon, Parfüm, Entschäumer ... | |

| | |
|---|---|
| ^{*)} Luviset^{®} Clear = Poly(Vinylpyrrolidon/Methcarylamid/Vinylimidazol) | |

Das Beispiel lässt sich mit den Polymeren der Beispiele 2 bis 20 wiederholen. Es wird jeweils ein VOC 55-Pumpspray mit guten Eigenschaften erhalten.

### Beispiel A11:

| Schaumfestiger | Gew.-% |
|---|---|
| | |
| Polymer aus Beispiel Nr. 1 | 10,00 |
| (30 %ige wässrig-ethanolische Dispersion) | |
| Luviset^{®} Clear ^{*)} (BASF AG) 20%ig | 5,00 |
| Cremophor^{®}A 25 (Ceteareth 25/Fa. BASF) | 0,20 |
| Comperlan^{®}KD (Coamide DEA/Fa. Henkel) | 0,10 |
| Wasser | 74,20 |
| Propan/Butan | 10,00 |
| weiterer Zusatz: Parfüm, Konservierungsmittel .... | |

### Herstellung: Einwiegen und unter Rühren lösen. Abfüllen und Treibgas zusetzen.

Das Beispiel lässt sich mit den Polymeren der Beispiele 2 bis 20 wiederholen. Es wird jeweils ein VOC 55-Pumpspray mit guten Eigenschaften erhalten.

### Beispiel A12:

| Haargel mit Aculyn^{®} 28: | Gew.-% |
|---|---|
| | |
| Phase 1: | |
| Polymer aus Beispiel Nr. 1 | 12,00 |
| (30 %ige wässrig-ethanolische Dispersion) | |
| Wasser, dest. | 37,00 |
| Aminomethylpropanol (38 %ige Lösung) | 1,0 |
| weiterer Zusatz: Konservierungsmittel, lösliches ethoxiliertes Silikon, Parfüm ... | |
| | |
| Phase 2: | |
| Aculyn^{®} 28 (1 %ige wässrige Suspension) | 50,00 |

Herstellung: Die Phasen 1 und 2 werden getrennt eingewogen und homogenisiert. Dann wird Phase 2 langsam in Phase 1 eingerührt. Es bildet sich ein fast klares, stabiles Gel.

Das Beispiel lässt sich mit den Polymeren der Beispiele 2 bis 20 wiederholen. Es wird jeweils ein Haargel mit Aculyn 28 mit guten Eigenschaften erhalten.

### Beispiel A13:

| Haargel mit Hydroxyethylcellulose | Gew.-% |
|---|---|
| | |
| Phase 1: | |
| Polymer aus Beispiel Nr. 1 | 12,00 |
| (30 %ige wässrig-ethanolische Dispersion) | |
| Wasser, dest. | 30,00 |
| weiterer Zusatz: Konservierungsmittel, lösliches ethoxiliertes Silikon, Parfüm ... | |
| | |
| Phase 2: | |
| Natrosol^{®} HR 250 (5 %ige Lösung) | 50,00 |
| Hydroxyethylcellulose (Fa. Hercules) | |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den Polymeren der Beispiele 2 bis 22 wiederholen. Es wird jeweils ein Haargel mit Hydroxyethylcellulose mit guten Eigenschaften erhalten.

### Beispiel A14:

| Conditionershampoo | Gew.-% |
|---|---|
| | |
| A) Texapon^{®} NSO 28 %ig | 50,00 |
| (Natriumlaurethsulfat/Fa. Henkel) | |
| Comperlan^{®} KS (Coamid DEA/Fa. Henkel) | 1,00 |
| Polymer aus Beispiel Nr. 14 | 12,00 |
| (30 %ige wässrig-ethanolische Dispersion) | |
| q. s. Parfümöl | |
| | |
| B) Wasser | 27,5 |
| Natriumchlorid | 1,5 |
| q- s. Konservierungsmittel ... | |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel. Das Beispiel lässt sich mit den Polymeren der Bei spiele 15 bis 22 wiederholen. Es wird jeweils ein Conditionershampoo mit guten Eigenschaften erhalten.

### Beispiel A15:

| Standard O/W-Creme: | | |
|---|---|---|
| | | |
| Ölphase: | Gew.-% | CTFA-Name |
| Cremophor^{®} A6 | 3,5 | Ceteareth-6 (and) Stearyl Alcohol |
| Cremophor^{®}A25 | 3,5 | Ceteareth-25 |
| Glycerinmonostearat s.e. | 2,5 | Glycerylstearat |
| Paraffinöl | 7,5 | Paraffin Oil |
| Cetylalkohol | 2,5 | Cetyl Alcohol |
| Luvitol^{®}EHO | 3,2 | Cetearyl Octanoate |
| Vitamin-E-Acetat | 1,0 | Toco pheryl Acetate |
| Nip-Nip | 0,1 | Meth yl- and Propyl-4-hydroxybenzoate |
| (7:3) | | |
| | | |

| Wasserphase: | | |
|---|---|---|
| | | |
| Polymer aus Beispiel Nr. 14 | 3,0 | |
| (30 %ige wässr.ethanol Dispersion) | | |
| Wasser | 74,6 | |
| 1,2-Propylenglykol | 1,5 | Propylenglykol |
| Germall^{®} II | 0,1 | Imidazolidinyl-Harnstoff |

### Herstellung:

Die Öl- und Wasserphasen werden getrennt eingewogen und bei einer Temperatur von ca. 80 °C homogenisiert. Dann wird die Wasserphase langsam in die Ölphase eingerührt und unter Rühren langsam auf Raumtemperatur abgekühlt.

Das Beispiel lässt sich mit den Polymeren der Be ispiele 15 bis 22 wiederholen. Es wird jeweils eine Standard O/W-Creme mit guten Eigenschaften erhalten.

### Beispiel A16:

### Sonnenschutz-Gel

| Gew.-% | |
|---|---|
| Phase A | |
| 1,00 | hydriertes Ricinusöl-PEG-40 |
| 8,00 | Octyl Methoxycinnamate (Uvinul^{®}MC 80 von BASF) |
| 5,00 | Octocrylene (Uvinul^{®}N 539 von BASF) |
| 0,80 | Octyl Triazone (Uvinul^{®}T 150 von BASF) |
| 2,00 | Butyl Methoxydibenzoylmethane (Uvinul^{®}BMBM von BASF) |
| 2,00 | Tocopherylacetat |
| q.s. | Parfümöl |
| | |

| Phase B | |
|---|---|
| 10,00 | Polymer 1 (30 %ige wässrig-ethanol. Dispersion) |
| 0,30 | Acrylat/C₁₀₋₃₀ Alkylacrylat-Copolymer |
| 0,20 | Carbomer |
| 5,00 | Glycerin |
| 0,20 | Dinatrium-EDTA |
| q.s. | Konservierungsmittel |
| 65,30 | dest. Wasser |
| | |

| Phase C | |
|---|---|
| 0,20 | Natriumhydroxid |

### Herstellung:

Die Komponenten der Phase A mischen. Phase B quellen lassen und unter Homogenisieren in Phase A einrühren. Mit Phase C neutralisieren und erneut homogenisieren.

Das Beispiel lässt sich mit den Polymeren der Beispiele 2 bis 22 wiederholen. Es wird jeweils ein Sonnenschutz-Gel mit guten Eigenschaften erhalten.

### Beispiel A17:

### Körperlotion-Schaum

| Gew.-% | |
|---|---|
| Phase A | |
| 1,50 | Ceteareth-25 |
| 1,50 | Ceteareth-6 |
| 4,00 | Cetearylalkohol |
| 10,00 | Cetearyloctanoat |
| 1,00 | Dimethicon |
| | |

| Phase B | |
|---|---|
| 3,00 | Polymer 1 (30 %ige wässrig-ethanol. Dispersion) |
| 2,00 | Panthenol |
| 2,50 | Propylenglykol |
| q.s. | Konservierungsmittel |
| 74,50 | dest. Wasser |
| | |

| Phase C | |
|---|---|
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Abfüllung: 90 % Wirkstoff und 10 % Propan/Butan bei 3,5 bar (20°C).

Das Beispiel lässt sich mit den Polymeren der Beispiele 2 bis 22 wiederholen. Es wird jeweils ein Körperlotion-Schaum mit guten Eigenschaften erhalten.

### Beispiel A18:

### Rasierschaum

| Gew.-% | |
|---|---|
| 6,00 | Ceteareth-25 |
| 5,00 | Poloxamer 407 |
| 52,00 | dest. Wasser |
| 1,00 | Triethanolamin |
| 5,00 | Propylenglykol |
| 1,00 | Lanolinöl-PEG-75 |
| 5,00 | erfindungsgemäßes Polymer 1 |
| | (30 %ige wässrig-etahnol. Dispersion) |
| q.s. | Konservierungsmittel |
| q.s. | Parfümöl |
| 25,00 | Sodium Laureth Sulfate |

### Herstellung:

Alles zusammen wiegen, danach rühren bis gelöst. Abfüllung: 90 Teile Wirksubstanz und 10 Teile Propan/Butan-Mischung 25:75.

Das Beispiel lässt sich mit den Polymeren der Beispiele 2 bis 22 wiederholen. Es wird jeweils ein Rasierschaum mit guten Eigenschaften erhalten.

### Herstellung:

Phase A lösen. Phase B in Phase A einstreuen und lösen. Phase C zugeben und unter Vakuum bei RT ca. 45 Min. rühren lassen.

### Beispiel A19:

### Duschgel

| Gew.-% | |
|---|---|
| 50,00 | Sodium Laureth Sulfate, Magnesium Laureth Sulfate, Sodium Laureth-8 Sulfate, Magnesium Laureth-8 |
| 1,00 | Cocoamide DEA |
| 4,00 | Polymer 1 (30 %ige wässrig-ethanol. Dispersion) |
| 2,00 | Sodium Laureth Sulfate, Glycol Distearate, Cocamide MEA, Laureth-10 |
| q.s. | Konservierungsmittel |
| q.s. | Parfümöl |
| 2,00 | Natriumchlorid |
| 41,00 | dest. Wasser |

### Herstellung:

Alle Komponenten gemeinsam einwiegen und bis zur Lösung rühren.

Das Beispiel lässt sich mit den Polymeren der Beispiele 2 bis 7 wiederholen. Es wird jeweils ein Duschgel mit guten Eigenschaften erhalten.

### Beispiel A20:

### Duschgel

| Gew.-% | |
|---|---|
| 30,00 | Sodium Laureth Sulfate |
| 6,00 | Sodium Cocoamphodiacetate |
| 6,00 | Cocamidopropylbetain |
| 3,00 | Sodium Laureth Sulfate, Glycol Distearate, Cocamide MEA, Laureth-10 |
| 7,70 | Polyquatemium-44 |
| 1,50 | Polymer 1 (25 %ige wässrige Dispersion) |
| 1,00 | Panthenol |
| q.s. | Konservierungsmittel |
| q.s. | Parfümöl |
| q.s. | Citronensäure |
| 0,50 | Natriumchlorid |
| 44,30 | dest. Wasser |

### Herstellung:

Die Komponenten der Phase A einwiegen und lösen. Den pH-Wert auf 6 bis 7 einstellen.

Das Beispiel lässt sich mit den Polymeren der Beispiele 2 bis 7 wiederholen. Es wird jeweils ein Duschgel mit guten Eigenschaften erhalten.

### Beispiel A20a:

### Duschbad

| Gew.-% | |
|---|---|
| A | |
| 40,00 | Sodium Laureth Sulfate |
| 5,00 | Sodium C₁₂₋₁₅ Pareth-15 Sulfonate |
| 5,00 | Decylglukosid |
| q.s. | Parfümöl |
| 0,10 | Phytantriol |
| | |

| B | |
|---|---|
| 35,80 | dest. Wasser |
| 0,1 | Guarhydroxypropyltrimoniumchlorid |
| 10,00 | Polymer 1 (30 %ige wässrig-ethanol. Dispersion) |
| 1,00 | Panthenol |
| q.s. | Konservierungsmittel |
| 1,00 | Laureth-3 |
| q.s. | Citronensäure |
| 2,00 | Natriumchlorid |

### Herstellung:

Die Komponenten der Phase A mischen. Die Komponenten der Phase B nacheinander zugeben und mischen. Den pH-Wert auf 6 bis 7 einstellen.

Das Beispiel lässt sich mit den Polymeren der Beispiele 2 bis 22 wiederholen. Es wird jeweils ein Duschbad mit guten Eigenschaften erhalten.

### Beispiel A21:

### Flüssigseife

| Gew.-% | |
|---|---|
| A | |
| 43,26 | dest. Wasser |
| 0,34 | Aminomethylpropanol |
| 3,40 | Poly(Ethylacrylat/Methacrylsäure) (Luviflex^{®}Soft, Fa. BASF) |
| | |

| B | |
|---|---|
| 40,00 | Sodium Laureth Sulfate |
| 10,00 | Cocamidopropylbetain |
| 1,00 | Polymer 1 (20 %ige wässrige Dispersion) |
| q.s. | Parfümöl |
| q.s. | Konservierungsmittel |
| 2,00 | Natriumchlorid |

### Herstellung:

Die Komponenten der Phase A einwiegen und klar lösen. Die Komponenten der Phase B nacheinander zugeben und mischen.

Das Beispiel lässt sich mit den Polymeren der Beispiele 2 bis 22 wiederholen. Es wird jeweils eine Flüssigseife mit guten Eigenschaften erhalten.

### Beispiel A22:

### Erfrischungsgel

| Gew.-% | |
|---|---|
| A | |
| 0,60 | Carbomer |
| 45,40 | dest. Wasser |
| | |

| B | |
|---|---|
| 0,50 | Bisabolol |
| 0,50 | Farnesol |
| q.s. | Parfümöl |
| 5,00 | PEG-40 Hydrogenated Castor Oil |
| 2,50 | Polymer 1 (30 %ige wässrig-ethanol. Dispersion) |
| 1,00 | Tetrahydroxypropylethylendiamin |
| 1,50 | Menthol |
| 43,00 | Alkohol |
| q.s. | C. I. 74 180, Direct Blue 86 |

### Herstellung:

Phase A quellen lassen. Phase B lösen. Phase B in Phase A einrühren.

Das Beispiel lässt sich mit den Polymeren der Beispiele 2 bis 22 wiederholen. Es wird jeweils ein Erfrischungsgel mit guten Eigenschaften erhalten.

### Beispiel A23:

### Aerosol-Haarschaum

| Gew.-% | |
|---|---|
| A | |
| 2,00 | Cocotrimoniummethsulfat |
| 0,20 | Parfümöl |
| | |

| B | |
|---|---|
| 63,90 | dest. Wasser |
| 6,70 | Polymer 1 (25 %ige wässrige Dispersion) |
| 0,50 | Poly(Ethylacrylat/Methacrylsäure) (Luviflex^{®}Soft, Fa. BASF) |
| 0,10 | Aminomethylpropanol |
| 0,20 | Ceteareth-25 |
| 0,20 | Trimethylsilylarnodimethicon, Trideceth-10, Cetrimonium Chloride |
| 0,10 | PEG-25 PABA |
| 0,20 | Hydroxyethylcellulose |
| 0,20 | PEG-8 |
| 0,20 | Panthenol |
| 15,00 | Alkohol |
| | |

| C | |
|---|---|
| 10,00 | Propan/Butan 3,5 bar (20 °C) |

### Herstellung:

Phasen A und B mischen und mit Treibgas abfüllen.

Das Beispiel lässt sich mit den Polymeren der Beispiele 2 bis 22 wiederholen. Es wird jeweils ein Aerosol-Haarschaum mit guten Eigenschaften erhalten.

### Beispiel A24:

### Pump-mousse

| Gew.-% | |
|---|---|
| A | |
| 2,00 | Cocotrimoniummethosulfat |
| q.s. | Parfümöl |
| | |

| C | |
|---|---|
| 74,30 | dest. Wasser |
| 7,00 | Polyquaternium-46 (10 %ige wässrige Lösung) |
| 15,00 | Polymer 1 (20 %ige wässrige Dispersion) |
| 0,50 | PEG-8 |
| 1,00 | Panthenol |
| q.s. | Konservierungsmittel |
| 0,20 | PEG-25 PABA (ethoxilierte p-Aminobenzoesäure) |

Herstellung: Die Komponenten der Phase A mischen. Die Komponenten der Phase B nacheinander zugeben und klar lösen.

Das Beispiel lässt sich mit den Polymeren der Beispiele 2 bis 7 wiederholen. Es wird jeweils ein Pumpmousse mit guten Eigenschaften erhalten.

### Beispiel A25:

### Aerosolschaum

| Gew.-% | |
|---|---|
| 10,00 | Polymer 1 (30 %ige wässrig-ethanol. Dispersion) |
| 5,00 | PVPNA-Copolymer (40 %ige wässrige Lösung) |
| 0,50 | Hydroxyethylcetyldimoniumphosphat |
| 0,20 | Ceteareth-25 |
| 0,40 | Parfümöl PC 910.781/Cremophor |
| 73,90 | dest. Wasser |
| q.s. | Konservierungsmittel |
| 10,00 | Propan/Butan 3,5 bar (20 °C) |

### Herstellung: Alles zusammen wiegen, rühren bis gelöst, dann abfüllen.

Das Beispiel lässt sich mit den Polymeren der Beispiele 2 bis 20 wiederholen. Es wird jeweils ein Aerosolschaum mit guten Eigenschaften erhalten.

### Beispiel A26:

### Farbstyling-Mousse

| Gew.-% | |
|---|---|
| A | |
| 2,00 | Cocotrimoniummethosulfat |
| q.s. | Parfümöl |
| | |

| B | |
|---|---|
| 20,00 | Polymer 14 (30 %ige wässrig-ethanol. Dispersion) |
| 0,50 | Acrylatcopolymer (Luvimer^{®}100 P, Fa. BASF) |
| 0,10 | Aminomethylpropanol |
| 0,20 | Ceteareth-25 |
| 0,20 | Panthenol |
| 0,20 | Hydroxyethylcellulose |
| 10,00 | Alkohol |
| 56,67 | dest. Wasser |
| 0,08 | C.I. 12245, Basic Red 76 |
| 0,05 | C.I. 42510, Basic Violet 14 |
| | |

| C | |
|---|---|
| 10,00 | Propan/Butan 3,5 bar (20 °C) |

Herstellung: Alles zusammen wiegen, rühren bis gelöst, dann abfüllen. Nur für dunkelblondes und braunes Haar geeignet!

Das Beispiel lässt sich mit den Polymeren der Beispiele 15 bis 20 wiederholen. Es wird jeweils ein Farbstyling-Mousse mit guten Eigenschaften erhalten.

### Beispiel A27:

### Pumphaarschaum

| Gew.-% | |
|---|---|
| A | |
| 1,50 | Cocotrimoniummethosulfat |
| q.s. | Parfümöl |
| | |

| B | |
|---|---|
| 10,00 | Polymer 1 (20 %ige wässrige Dispersion) |
| 86,04 | dest. Wasser |

| | |
|---|---|
| C | |
| 0,46 | Amino methylpropanol |
| 4,00 | PEG/PPG-25/25 Dimethicon/Acrylat-Copolymer |
| q.s. | Konservierungsmittel |

Herstellung: Phase A mischen. Phase B in Phase A einrühren. Phase C zugeben und rühren bis gelöst.

Das Beispiel lässt sich mit den Polymeren der Beispiele 2 bis 22 wiederholen. Es wird jeweils ein Pumphaarschaum mit guten Eigenschaften erhalten.

### Beispiel A28:

### Aquawax

| Gew.-% | |
|---|---|
| 50,00 | Polymer 1 (20 %ige wässrige Dispersion) |
| q.s. | Parfümöl |
| q.s. | hydriertes Rizinusöl-PEG-40 |
| 0,10 | Diethylphthalat |
| 0,10 | Cetearylethylhexanoat |
| 0,10 | PEG-7 Glyceryl Cocoate |
| 0,10 | Konservierungsmittel |
| 47,70 | dest. Wasser |
| 2,00 | Capryl/Caprin-Triglycerid, Acrylat-Copolymer |

Herstellung: Alles mischen und homogenisieren. 15 Minuten nachrühren.

Das Beispiel lässt sich mit den Polymeren der Beispiele 2 bis 22 wiederholen. Es wird jeweils ein Aquawax mit guten Eigenschaften erhalten.

### Beispiel A29 :

### Shampoo

| Gew.-% | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glycol Distearate, Cocamide MEA, Laureth-10 |
| 3,00 | Polymer 1 (30 %ige wässrig-ethanol. Dispersion) |
| 2,00 | Dimethicon |
| q.s. | Parfum |
| q.s. | Konservierungsstoff |
| q.s. | Citronensäure |
| 1,00 | Natriumchlorid |
| ad 100 | dest. Wasser |

### Herstellung:

### Komponenten einwiegen und lösen. pH-Wert auf 6 bis 7 einstellen.

Das Beispiel lässt sich mit den Polymeren der Beispiele 2 bis 22 wiederholen. Es wird jeweils ein Shampoo mit guten Eigenschaften erhalten.

### Beispiel A30:

### Antischuppen-Shampoo

| Gew.-% | |
|---|---|
| 40,00 | Sodium Laureth Sulfate |
| 10,00 | Cocamidopropylbetain |
| 10,00 | Disodium Laureth Sulfosuccinate |
| 2,50 | Sodium Laureth Sulfate, Glycol Distearate, Cocamide MEA, Laureth-10 |
| 3,00 | Polymer 1 ((30 %ige wässrig-ethanol. Dispersion) |
| 0,50 | Climbazol |
| q.s. | Parfum |
| q.s. | Konservierungsstoff |
| 0,50 | Natriumchlorid |
| ad 100 | dest. Wasser |

### Herstellung:

### Komponenten einwiegen und lösen. pH-Wert auf 6 bis 7 einstellen.

Das Beispiel lässt sich mit den Polymeren der Beispiele 2 bis 22 wiederholen. Es wird jeweils ein Antischuppen-Shampoo mit guten Eigenschaften erhalten.

### Beispiel A31:

### Klares Duschgel

| Gew.-% | |
|---|---|
| 40,00 | Sodium Laureth Sulfate |
| 5,00 | Decylglukosid |
| 5,00 | Cocamidopropylbetain |
| 3,00 | Polymer 14 (30 %ige wässrig-ethanol. Dispersion) |
| 1,00 | Panthenol |
| q.s. | Parfum |
| q.s. | Konservierungsstoff |
| q.s. | Citronensäure |
| 2,00 | Natriumchlorid |
| ad 100 | dest. Wasser |

### Herstellung:

### Komponenten einwiegen und lösen. pH-Wert auf 6 bis 7 einstellen.

Das Beispiel lässt sich mit den Polymeren der Beispiele 15 bis 22 wiederholen. Es wird jeweils ein klares Duschgel mit guten Eigenschaften erhalten.

## Patentansprüche

1. Copolymer, erhältlich durch radikalische Polymerisation eines Monomergemisches M enthaltend
a) 30 bis 80 Gew.-% Ethylmethacrylat oder 30 bis 80 Gew.-% eines Gemisches aus Ethylmethacrylat und wenigstens einer von Ethylmethacrylat unterschiedlichen Verbindung der allgemeinen Formel I 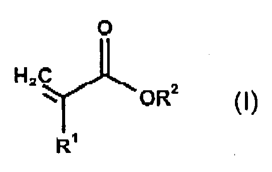 worin
R¹ für H oder CH₃ steht,
R² für C₁-C₄-Alkyl steht,
b) 0 bis 40 Gew.-% mindestens einer N-Vinyllactam-Verblndung,
c) 5 bis 35 Gew.-% mindestens einer monoethylenisch ungesättigten Carbonsäure,
d) 0.1 bis 30 Gew.-% mindestens einer Verbindung mit einer radikalisch polymerisierbaren, alpha,beta-ethylenisch ungesättigten Doppelbindung und wenigstens einer kationogenen und/oder kationischen Gruppe pro Molekül,
e) 0 bis 20 Gew.-% gegebenenfalls welterer, von a), b), c) und d) verschiedener, radikalisch polymerisierbarer Monomere, wobei sich die Anteile der Komponenten a) bis e) zu 100 Gew.-% addieren und wobei das Monomergemisch M wenigstens 20 Gew.-% Ethylmethacrylat enthält, und wobei
das Verhältnis der molaren Gesamtmenge der Carboxyl- und Carboxylat-Gruppen zur molaren Gesamtmenge der kationogenen und kationischen Gruppen wenigstens 2 beträgt.

2. Copolymer nach Anspruch 1, wobei a) Ethylmethacrylat oder ein Gemisch umfassend oder bestehend aus Ethylmethacrylat und tert-Butylacrylat ist.

3. Copolymer nach einem der Ansprüche 1 bis 2, wobei b) ausgewählt ist aus der Gruppe bestehend aus N-Vinylpyrrolidon, N-Vinylpiperidon, N-Vinylcaprolactam, N-Viny1-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam und deren Mischungen.

4. Copolymer nach einem der Ansprüche 1 bis 3, wobei c) aus Methacrylsäure oder einer Mischung von Methacrylsäure mit wenigstens einer weiteren monoethylenisch ungesättigten Carbonsäure besteht.

5. Copolymer nach einem der Ansprüche 1 bis 4, wobei c) aus Methacrylsäure oder einer Mischung aus Methacrylsäure und Acrylsäure besteht.

6. Copolymer nach einem der Ansprüche 4 oder 5, wobei das Gewichtsverhältnis von Methacrylsäure zu den weiteren monoethylenisch ungesättigten Carbonsäuren wenigstens größer als 1, bevorzugt größer als 2 ist.

7. Copolymer nach einem der Ansprüche 1 bis 6, wobei d) ausgewählt ist aus der Gruppe der Verbindungen mit einer radikalisch polymerisierbaren, alpha,beta-ethylenisch ungesättigten Doppelbindung und wenigstens einer Amino- und/oder Ammoniumgruppe.

8. Copolymer nach Anspruch 7, wobei die wenigstens eine Aminogruppe der Komponente d) ausgewählt ist aus primären, sekundären und tertlären Aminogruppen.

9. Copolymer nach einem der Ansprüche 1 bis 8, wobei d) ausgewählt ist aus der Gruppe bestehend aus
d1) Estern alpha,beta-ethylenisch ungesättigter Mono- und Dicarbonsäuren mit Aminoalkoholen, welche am Aminstickstoff mono- oder dialkyliert sein können,
d2) Amiden □alpha,beta-ethylenisch ungesättigter Mono- und Dicarbonsäuren mit Diaminen, welche mindestens eine primäre oder sekundäre Aminogruppe aufweisen,
d3) N,N-Diallylamin,
d4) N,N-Diallyl-N-alkylaminen und deren Derivaten,
d5) vinyl- und allylsubstituierten Stickstoffheterocyclen,
d6) vinyl- und allylsubstituierten heteroaromatischen Verbindungen
und deren Mischungen.

10. Copolymer nach einem der Ansprüche 1 bis 9 erhältlich durch radikalische Polymerisation eines Monomergemisches M enthaltend
a) 30 bis 70 Gew.-% Komponente a)
b) 10 bis 40 Gew.-% Komponente b)
c) 10 bis 25 Gew.-% Komponente c)
d) 0.5 bis 5 Gew.-% Komponente d).

11. Copolymer nach einem der Ansprüche 1 bis 9 erhältlich durch radikalische Polymerisation eines Monomergemisches M enthaltend
a) 50 bis 80 Gew.-% Komponente a)
b) 0 Gew.-% Komponente b)
c) 15 bis 35 Gew.-% Komponente c)
d) 0,5 bis 12 Gew.-% Komponente d).

12. Copolymer nach einem der Ansprüche 1 bis 11. wobei
a) Ethylmethacrylat,
b) N-Vinylpyrrolidon oder N-Vinylcaprolactam oder deren Mischung,
c) Methacrylsäure oder eine Mischung aus Methacrylsäure und Acrylsäure und
d) Monomer ausgewählt aus der Gruppe bestehend aus N-[3-(dimethylamino)propyl](meth)acrylamid, N-(tert.-Butyl)aminoethylmethacrylat, Vinylimidazol und deren Mischungen ist.

13. Kosmetisches oder pharmazeutisches Mittel, enthaltend
A) wenigstens ein Copolymer gemäß einem der Ansprüche 1 bis 12 und
B) wenigstens einen kosmetisch oder pharmazeutisch akzeptablen Träger.

14. Mittel nach Anspruch 13 wobei die Komponente B) ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen.
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen
und Mischungen davon.

15. Mittel nach einem der Ansprüche 13 oder 14, enthaltend wenigstens einen von den Komponenten A) und B) verschiedenen Zusatzstoff, der ausgewählt ist unter kosmetisch aktiven, Wirkstoffen, Emulgatoren, Tensiden, Konservierungsmitteln, Parfümölen. Verdickern, Haarpolymeren, Haar- und Hautconditionern, Pfropfpolymeren, wasserlöslichen oder dispergierbaren silikonhaltigen Polymeren. Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollentien und Weichmachern.

16. Mittel nach einem der Ansprüche 13 bis 15, enthaltend zusätzlich wenigstens ein nichtionisches, ein anionisches oder ein ampholytisches Polymer.

17. Mittel nach Anspruch 16, wobei das anionische Polymer ein Polyurethan ist.

18. Mittel nach einem der Ansprüche 13 bis 17, wobei im Mittel enthaltene Säuregruppen, insbesondere Carboxylgruppen, mindestens teilweise durch Aminogruppen enthaltende Silikonpolymere neutralisiert sind.

19. Mittel nach einem der Ansprüche 13 bis 18 in Form eines Gels, Schaums, Sprays, einer Mousse, Salbe, Creme, Emulsion, Suspension. Lotion, Milch oder Paste.

20. Mittel nach einem der Ansprüche 13 bis 19, wobei das Mittel einen Anteil an flüchtigen organischen Komponenten von höchstens 80 Gew.-%, bevorzugt höchstens 55 Gew.-% besitzt.

21. Verwendung eines Copolymers gemäß einem der Ansprüche 1 bis 12 in Hautreinigungsmitteln, Mitteln zur Pflege und zum Schutz der Haut, Nagelpflegemitteln, Zubereltungen für die dekorative Kosmetik und Haarbehandlungsmittein.

22. Verwendung nach Anspruch 21 in Haarbehandlungsmittein als Festiger und/oder als Conditioner.

23. Verwendung nach Anspruch 22, wobei das Mittel in Form eines Haargels, Shampoos, Schaumfestigers, Haarwassers, Haarsprays oder Haarschaums vorliegt.

24. Verwendung eines Copolymers gemäß einem der Ansprüche 1 bis 12 als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

25. Verfahren zur Herstellung der Copolymere gemäß mindestens einem der Ansprüche 1 bis 12 umfassend die Schritte
a) radikalische Polymerisation des Monomergemisches M enthaltend die Komponenten a), b), c), d) und gegebenenfalls e) bei einem pH-Wert im Bereich von 5 bis 8, bevorzugt 6,5 bis 7,5,
b) Einstellen des pH-Wertes nach abgeschlossener Polyerisation auf einen Wert im Bereich von 5 bis 7, bevorzugt 5,5 bis 6,5,
c) Wasserdampf-Destillation der Polymerisationslösung.
d) gegebenenfalls Einstellung des pH-Wertes der destillierten Polymerisationslösung auf einen Wert im Bereich von 7,5 bis 9,0, bevorzugt 8,0. bis 8,5 nach der Wasserdampfdestillation.

## Claims

1. A copolymer obtainable by free-radical polymerization of a monomer mixture M comprising
a) 30 to 80% by weight of ethyl methacrylate or 30 to 80% by weight of a mixture of ethyl methacrylate and at least one compound of the general formula I different from ethyl methacrylate 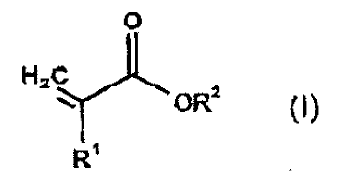 in which
R¹ is H or CH₃,
R² is C₁-C₄-alkyl,
b) 0 to 40% by weight of at least one N-vinyllactam compound,
c) 5 to 35% by weight of at least one monoethylenically unsaturated carboxylic acid,
d) 0.1 to 30% by weight of at least one compound with a free-radically polymerizable, alpha,beta-ethylenically unsaturated double bond and at least one cationogenic and/or cationic group per molecule,
e) 0 to 20% by weight if appropriate of further free-radically polymerizable monomers different from a) , b), c) and d), where the fractions of components a) to e) add up to 100% by weight and where the monomer mixture M comprises at least 20% by weight of ethyl methacrylate, and where the ratio of the total molar amount of the carboxyl and carboxylate groups to the total molar amount of the cationogenic and cationic groups is at least 2.

2. The copolymer according to claim 1, where a) is ethyl methacrylate or a mixture comprising or consisting of ethyl methacrylate and tert-butyl acrylate.

3. The copolymer according to one of claims 1 to 2, where b) is chosen from the group consisting of N-vinylpyrrolidone, N-vinylpiperidone, N-vinylcaprolactam, N-vinyl-5-methyl-2-pyrrolidone, N-vinyl-5-ethyl-2-pyrrolidone, N-vinyl-6-methyl-2-piperidone, N-vinyl-6-ethyl-2-piperidone, N-vinyl-7-methyl-2-caprolactam, N-vinyl-7-ethyl-2-caprolactam and mixtures thereof.

4. The copolymer according to one of claims 1 to 3, where c) consists of methacrylic acid or a mixture of methacrylic acid with at least one further monoethylenically unsaturated carboxylic acid.

5. The copolymer according to one of claims 1 to 4, where c) consists of methacrylic acid or a mixture of methacrylic acid and acrylic acid

6. The copolymer according to one of claims 4 or 5, where the weight ratio of methacrylic acid to the further monoethylenically unsaturated carboxylic acids is at least greater than 1, preferably greater than 2.

7. The copolymer according to one of claims 1 to 6, where d) is chosen from the group of compounds with a free-radically polymerizable, alpha,beta-ethylenically unsaturated double bond and at least one amino and/or ammonium group.

8. The copolymer according to claim 7, where the at least one amino group of component d) is chosen from primary, secondary and tertiary amino groups.

9. The copolymer according to one of claims 1 to 8, where d) is chosen from the group consisting of
d1) esters of alpha,beta -ethylenically unsaturated mono- and dicarboxylic acids with amino alcohols which may be mono- or dialkylated on the amine nitrogen,
d2) amides of alpha,beta -ethylenically unsaturated mono- and dicarboxylic acids with diamines which have at least one primary or secondary amino group,
d3) N,N-diallylamine,
d4) N,N-diallyl-N-alkylamines and derivatives thereof,
d5) vinyl- and allyl-substituted nitrogen heterocycles,
d6) vinyl- and allyl-substituted heteroaromatic compounds
and mixtures thereof.

10. The copolymer according to one of claims 1 to 9 obtainable by free-radical polymerization of a monomer mixture M comprising
a) 30 to 70% by weight of component a)
b) 10 to 40% by weight of component b)
c) 10 to 25% by weight of component c)
d) 0.5 to 5% by weight of component d).

11. The copolymer according to one of claims 1 to 9 obtainable by free-radical polymerization of a monomer mixture M comprising
a) 50 to 80% by weight of component a)
b) 0% by weight of component b)
c) 15 to 35% by weight of component c)
d) 0.5 to 12% by weight of component d).

12. The copolymer according to one of claims 1 to 11, where
a) is ethyl methacrylate,
b) is N-vinylpyrrolidone or N-vinylcaprolactam or mixture thereof,
c) is methacrylic acid or a mixture of methacrylic acid and acrylic acid and
d) is monomer chosen from the group consisting of N-[3-(dimethylamino)propyl](meth)acrylamide, N-(tert-butyl)aminoethyl methacrylate, vinylimidazole and mixtures thereof.

13. A cosmetic or pharmaceutical composition comprising
A) at least one copolymer according to one of claims 1 to 12 and
B) at least one cosmetically or pharmaceutically acceptable carrier.

14. The composition according to claim 13, where the component B) is chosen from
i) water,
ii) water-miscible organic solvents, preferably C₂-C₄-alkanols, in particular ethanol,
iii) oils, fats, waxes,
iv) esters of C₆-C₃₀-monocarboxylic acids with mono-, di- or trihydric alcohols which are different from iii),
v) saturated acyclic and cyclic hydrocarbons,
vi) fatty acids,
vii) fatty alcohols,
viii)propellant gases
and mixtures thereof.

15. The composition according to one of claims 13 or 14, comprising at least one additive different from the components A) and B) which is chosen from cosmetically active ingredients, emulsifiers, surfactants, preservatives, perfume oils, thickeners, hair polymers, hair and skin conditioners, graft polymers, water-soluble or dispersible silicone-containing polymers, photoprotective agents, bleaches, gel formers, care agents, colorants, tinting agents, tanning agents, dyes, pigments, bodying agents, humectants, regreasing agents, collagen, protein hydrolyzates, lipids, antioxidants, antifoams, antistats, emollients and softeners.

16. The composition according to one of claims 13 to 15, additionally comprising at least one nonionic, one anionic or one ampholytic polymer.

17. The composition according to claim 16, where the anionic polymer is a polyurethane.

18. The composition according to one of claims 13 to 17, where acid groups, in particular carboxyl groups, present in the composition are at least partially neutralized by silicone polymers comprising amino groups.

19. The composition according to one of claims 13 to 18 in the form of a gel, foam, spray, mousse, ointment, cream, emulsion, suspension, lotion, milk or paste.

20. The composition according to one of claims 13 to 19, where the composition has a fraction of volatile organic components of at most 80% by weight, preferably at most 55% by weight.

21. The use of a copolymer according to one of claims 1 to 12 in skin-cleansing compositions, compositions for the care and protection of the skin, nailcare compositions, preparations for decorative cosmetics and hair-treatment compositions.

22. The use according to claim 21 in hair-treatment compositions as setting agents and/or as conditioners.

23. The use according to claim 22, where the composition is in the form of a hair gel, shampoo, setting foam, hair tonic, hair spray or hair foam.

24. The use of a copolymer according to one of claims 1 to 12 as auxiliary in pharmacy, preferably as or in (a) coating(s) for solid drug forms, for modifying rheological properties, as surface-active compound, as or in (an) adhesive (s) , and as or in (a) coating(s) for the textile, paper, printing and leather industry.

25. A method of preparing copolymers according to at least one of claims 1 to 12 comprising the steps
a) free-radical polymerization of the monomer mixture M comprising the components a), b), c), d) and if appropriate e) at a pH in the range from 5 to 8, preferably 6.5 to 7.5,
b) adjustment of the pH when polymerization is complete to a value in the range from 5 to 7, preferably 5.5 to 6.5,
c) steam distillation of the polymerization solution,
d) if appropriate adjustment of the pH of the distilled polymerization solution to a value in the range from 7.5 to 9.0, preferably 8.0 to 8.5 after the steam distillation.

## Revendications

1. Copolymère, pouvant être obtenu par polymérisation radicalaire d'un mélange de monomères M, contenant
a) 30 à 80% en poids de méthacrylate d'éthyle ou 30 à 80% en poids d'un mélange de méthacrylate d'éthyle et d'au moins un composé différent du méthacrylate d'éthyle de formule générale I 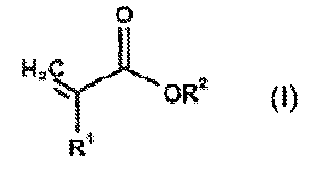 où
R¹ représente hydrogène ou CH₃,
R² représente C₁-C₄-alkyle,
b) 0 à 40% en poids d'au moins un composé de type N-vinyllactame,
c) 5 à 35% en poids d'au moins un acide carboxylique éthyléniquement monoinsaturé,
d) 0,1 à 30% en poids d'au moins un composé présentant une double liaison polymérisable par voie radicalaire, alpha,bêta-éthyléniquement insaturée et au moins un groupe cationogène et/ou cationique par molécule,
e) 0 à 20% en poids le cas échéant d'autres monomères, différents de a), b), c) et d), polymérisables par voie radicalaire, la proportion des composants a) à e) s'ajoutant jusqu'à 100% en poids et le mélange de monomères M contenant au moins 20% en poids de méthacrylate d'éthyle et
le rapport de la quantité molaire totale des groupes carboxyle et carboxylate à la quantité molaire totale des groupes cationogènes et cationiques étant d'au moins 2.

2. Copolymère selon la revendication 1, où a) est le méthacrylate d'éthyle ou un mélange comprenant ou constitué par du méthacrylate d'éthyle et de l'acrylate de tert-butyle.

3. Copolymère selon l'une quelconque des revendications 1 à 2, b) étant choisi dans le groupe formé par la N-vinylpyrrolidone, la N-vinylpipéridone, le N-vinylcaprolactame, la N-vinyl-5-méthyl-2-pyrrolidone, la N-vinyl-5-éthyl-2-pyrrolidone, la N-vinyl-6-méthyl-2-pipéridone, la N-vinyl-6-éthyl-2-pipéridone, le N-vinyl-7-méthyl-2-caprolactame, le N-vinyl-7-éthyl-2-caprolactame et leurs mélanges.

4. Copolymère selon l'une quelconque des revendications 1 à 3, c) étant constitué par l'acide méthacrylique ou un mélange d'acide méthacrylique avec au moins un autre acide carboxylique éthyléniquement monoinsaturé.

5. Copolymère selon l'une quelconque des revendications 1 à 4, c) étant constitué par l'acide méthacrylique ou un mélange d'acide méthacrylique et d'acide acrylique.

6. Copolymère selon l'une quelconque des revendications 4 ou 5, le rapport pondéral de l'acide méthacrylique aux autres acides carboxyliques éthyléniquement monoinsaturés étant au moins supérieur à 1, de préférence supérieur à 2.

7. Copolymère selon l'une quelconque des revendications 1 à 6, d) étant choisi dans le groupe des composés présentant une double liaison polymérisable par voie radicalaire, alpha,bêta-éthyléniquement insaturée et au moins un groupe amino et/ou ammonium.

8. Copolymère selon la revendication 7, ledit au moins un groupe amino du composant d) étant choisi parmi les groupes amino primaires, secondaires et tertiaires.

9. Copolymère selon l'une quelconque des revendications 1 à 8, où d) est choisi dans le groupe constitué par
d1) les esters d'acides monocarboxyliques et dicarboxyliques alpha,beta-éthyléniquement insaturés avec des aminoalcools, qui peuvent être monoalkylés ou dialkylés sur l'azote de la fonction amine,
d2) les amides d'acides monocarboxyliques et dicarboxyliques alpha,beta-éthyléniquement insaturés avec des diamines, qui présentent au moins un groupe amino primaire ou secondaire,
d3) la N,N-diallylamine,
d4) les N,N-diallyl-N-alkylamines et leurs dérivés,
d5) les hétérocycles azotés substitués par vinyle et allyle,
d6) les composés hétéroaromatiques azotés substitués par vinyle et allyle,
et leurs mélanges.

10. Copolymère selon l'une quelconque des revendications 1 à 9, pouvant être obtenu par polymérisation radicalaire d'un mélange de monomères M, contenant
a) 30 à 70% en poids de composant a)
b) 10 à 40% en poids de composant b)
c) 10 à 25% en poids de composant c)
d) 0,5 à 5% en poids de composant d).

11. Copolymère selon l'une quelconque des revendications 1 à 9, pouvant être obtenu par polymérisation radicalaire d'un mélange de monomères M, contenant
a) 50 à 80% en poids de composant a)
b) 0% en poids de composant (b)
c) 15 à 35% en poids de composant c)
d) 0,5 à 12% en poids de composant d).

12. Copolymère selon l'une quelconque des revendications 1 à 11, où
a) est du méthacrylate d'éthyle,
b) est de la N-vinylpyrrolidone ou du N-vinylcaprolactame ou leur mélange,
c) est de l'acide méthacrylique ou un mélange d'acide méthacrylique et d'acide acrylique et
d) est un monomère choisi dans le groupe constitué par le N-[3-(diméthylamino)propyl](méth)acrylamide, le méthacrylate de N-(tert-butyl)aminoéthyle, le vinylimidazole et leurs mélanges.

13. Agent cosmétique ou pharmaceutique, contenant
A) au moins un copolymère selon l'une quelconque des revendications 1 à 12 et
B) au moins un support cosmétiquement ou pharmaceutiquement acceptable.

14. Agent selon la revendication 13, le composant B) étant choisi parmi
i) l'eau
ii) les solvants organiques miscibles à l'eau, de préférence les C₂-C₄-alcanols, en particulier l'éthanol,
iii) les huiles, les graisses, les cires,
iv) les esters différents de iii) d'acides monocarboxyliques en C₆-C₃₀ avec des alcools monovalents, divalents ou trivalents,
v) les hydrocarbures acycliques et cycliques saturés,
vi) les acides gras,
vii) les alcools gras,
viii) les gaz propulseurs
et leurs mélanges.

15. Agent selon l'une quelconque des revendications 13 ou 14, contenant au moins un additif différent des composants A) et B) qui est choisi parmi les substances cosmétiquement actives, les émulsifiants, les agents tensioactifs, les agents de conservation, les huiles parfumées, les épaississants, les polymères pour cheveux, les agents de conditionnement pour cheveux et la peau, les polymères greffés, les polymères siliconés, solubles ou dispersibles dans l'eau, les agents de protection contre la lumière, les agents de blanchiment, les agents de formation de gel, les agents de soin, les agents de coloration, les agents de teinture, les agents de bronzage, les colorants, les pigments, les agents conférant une consistance, les humectants, les relipidants, le collagène, les hydrolysats de protéines, les lipides, les antioxydants, les antimousses, les antistatiques, les émollients et les plastifiants.

16. Agent selon l'une quelconque des revendications 13 à 15, contenant au moins un polymère non ionique, anionique ou ampholytique.

17. Agent selon la revendication 16, le polymère anionique étant un polyuréthane.

18. Agent selon l'une quelconque des revendications 13 à 17, les groupes acides contenus dans l'agent, en particulier les groupes carboxyle, étant neutralisés au moins partiellement par des polymères siliconés contenant des groupes amino.

19. Agent selon l'une quelconque des revendications 13 à 18 sous forme d'un gel, d'une mousse, d'un spray, d'une pommade, d'une crème, d'une émulsion, d'une suspension, d'une lotion, d'un lait ou d'une pâte.

20. Agent selon l'une quelconque des revendications 13 à 19, l'agent présentant une proportion de composants organiques volatils d'au maximum 80% en poids, de préférence d'au maximum 55% en poids.

21. Utilisation d'un copolymère selon l'une quelconque des revendications 1 à 12 dans les agents de nettoyage de la peau, les agents pour le soin et la protection de la peau, les agents de soin des ongles, les préparations pour la cosmétique décorative et les agents de traitement des cheveux.

22. Utilisation selon la revendication 21 dans les agents de traitement des cheveux comme fixateurs et/ou comme agents de conditionnement.

23. Utilisation selon la revendication 22, l'agent se trouvant sous forme d'un gel pour cheveux, d'un shampooing, d'un fixateur en mousse, d'une lotion capillaire, d'un spray pour cheveux ou d'une mousse pour cheveux.

24. Utilisation d'un copolymère selon l'une quelconque des revendications 1 à 12 comme adjuvant en pharmacie, de préférence comme ou dans (les) agent(s) de revêtement pour les formes médicamenteuses solides, pour la modification des propriétés rhéologiques, comme composé tensioactif, comme ou dans (les) adhésif(s) ainsi que comme ou dans (les) agent(s) de revêtement pour l'industrie textile, du papier, de l'impression et du cuir.

25. Procédé pour la préparation des copolymères selon au moins l'une quelconque des revendications 1 à 12, comprenant les étapes de
a) polymérisation radicalaire du mélange de monomères M contenant les composants a), b), c), d) et le cas échéant e) à un pH dans la plage de 5 à 8, de préférence de 6,5 à 7,5.
b) réglage du pH après la fin de la polymérisation à une valeur dans la plage de 5 à 7, de préférence de 5,5 à 6,5.
c) distillation à la vapeur d'eau de la solution de polymérisation.
d) le cas échéant réglage du pH de la solution de polymérisation distillée à une valeur dans la plage de 7,5 à 9,0, de préférence de 8,0 à 8,5 après la distillation à la vapeur d'eau.
